# EUROPEAN PATENT SPECIFICATION

(11) **EP 2 536 753 B1**
(45) Date of publication and mention of the grant of the patent: **20.12.2017**
(21) Application number: 11701527.1
(22) Date of filing: 02.02.2011
(51) Int. Cl.: C07K 14/755, A61K 38/37

(54) **FACTOR VIII MOLECULES WITH REDUCED VWF BINDING**
Faktor-VIII-Moleküle mit reduzierter VWF-Bindung
Molécules de facteur VIII avec liaison VWF réduite

(30) Priority: 18.02.2010 US 305608 P; 16.02.2010 EP 10153718
(43) Date of publication of application: 26.12.2012
(73) Proprietor: Novo Nordisk A/S, 2880 Bagsværd (DK)
(72) Inventor: PESCHKE, Bernd, DK-2760 Måløv (DK); KOFOD-HANSEN, Mikael, DK-2200 København N (DK); BUCHARDT, Jens, DK-2820 Gentofte (DK); STENNICKE, Henning Ralf, DK-2980 Kokkedal (DK); ØSTERGAARD, Henrik, DK-3650 Ølstykke (DK); KJALKE, Marianne, DK-3600 Frederikssund (DK); OLSEN, Eva H. Norling, DK-2750 Ballerup (DK); HANSEN, Jens Jacob, DK-4040 Jyllinge (DK)
(86) International application number: PCT/EP2011/051438
(87) International publication number: WO 2011/101242

(56) References cited:
- WO-A1-2009/108806
- WO-A2-2006/103298
- THIM L ET AL: "Purification and characterization of a new recombinant factor VIII (N8)", HAEMOPHILIA 2010 BLACKWELL PUBLISHING LTD GBR LNKD- DOI:10.1111/J.1365-2516.2009.02135.X, vol. 16, no. 2, 11 November 2009 (2009-11-11), pages 349-359, XP002583862,
- "Abstracts from XXII ISTH congress", JOURNAL OF THROMBOSIS AND HAEMOSTASIS, vol. 7, no. supplement 2, July 2009 (2009-07), pages 508-517, XP002583863,
- HIGUCHI M ET AL: "Characterization of mutations in the factor VIII gene by direct sequencing of amplified genomic DNA", GENOMICS, ACADEMIC PRESS, SAN DIEGO, US LNKD- DOI:10.1016/0888-7543(90)90448-4, vol. 6, no. 1, 1 January 1990 (1990-01-01) , pages 65-71, XP024797421, ISSN: 0888-7543 [retrieved on 1990-01-01]

## Description

### FIELD OF THE INVENTION

The present invention relates to recombinant factor VIII (FVIII) molecules. In particular, the present invention relates to FVIII molecules having reduced von Willebrand factor (vWF) binding compared to endogenous FVIII. The invention furthermore relates to use of such molecules as well as methods for obtaining such molecules.

### BACKGROUND OF THE INVENTION

Haemophilia A is an inherited bleeding disorder caused by deficiency or dysfunction of coagulation factor VIII (FVIII) activity. The clinical manifestation is not on primary haemostasis - formation of the blood clot occurs normally - but the clot is unstable due to a lack of secondary thrombin formation. The disease is treated by intravenously injection of coagulation factor FVIII which is either isolated from blood or produced recombinantly.

Current treatment recommendations are moving from traditional on-demand treatment towards prophylaxis. The circulatory half life of endogenous FVIII bound to von Willebrandt Factor is 12-14 hours and prophylactic treatment is thus to be performed several times a week in order to obtain a virtually symptom-free life for the patients. IV administration is for many, especially children and young persons, associated with significant inconvenience and/or pain. There is thus a need in the art for novel Factor VIII products with factor VIII activity that are preferably homogenous in structure, preferably safe and preferably have a significantly prolonged circulatory half life in order to reduce the number of factor VIII administration per week. There is furthermore a need in the art for relatively simple methods for obtaining and producing such molecules.

WO2009108806 discloses conjugated FVIII molecules - such molecules may furthermore have modifications that may modulate binding capacity of FVIII with various other cell components.

Higuchi et al Genomics, Academic Press, San Diego, US DOI:10.1016/0888-7543(90)90448-4, vol. 6, no. 1, 1 January 1990, pages 65-71 disclose investigations of genomic DNA from 127 patients with hameophilia A. Various mutations are disclosed herein, including Y1680F.

### SUMMARY OF THE INVENTION

The present invention relates to a recombinant Factor VIII molecule, wherein said molecule has prolonged circulatory half-life and reduced vWF binding capacity, and wherein said molecule is covalently conjugated with at least one side group such that said side group, is selected from one or more of the group consisting of: hydrophilic polymer, an albumin binder, an antibody or a fragment thereof, and albumin; and wherein the molecule comprises a point mutation and/or a deletion in the region spanning amino acid residues 1670-1684 in SEQ ID NO: 1. The invention furthermore relates to methods for making such molecules as well as use of such molecules. Such molecules are having a modified circulatory half life.

### DETAILED DESCRIPTION OF THE INVENTION

### Brief description of drawings:

- **Figure 1**: Dose response relationship for NNC129-0000-9105 and Advate® in the FeCl₃ induced injury model in FVIII-KO mice (n=6-10). *significantly different from vehicle treatment. *P < 0.05.
- **Figure 2**: Effect of NNC0129-000-9105 and Advate® (20 and 280 IU/kg) in the tail bleeding model in FVIII-KO mice (n=5-6) *significantly different from vehicle treatment.*P < 0.05.

### Definitions:

Von Willebrandt Factor (vWF'): vWF is a large mono-/multimeric glycoprotein present in blood plasma and produced constitutively in endothelium (in the Weibel-Palade bodies), megakaryocytes (α-granules of platelets), and subendothelial connective tissue. Its primary function is binding to other proteins, particularly Factor VIII and it is important in platelet adhesion to wound sites.

Factor VIII is bound to vWF while inactive in circulation; Factor VIII degrades rapidly or is cleared when not bound to vWF. It thus follows that reduction or abolishment of vWF binding capacity in FVIII has thus far been considered as a highly undesirable approach in obtaining Factor FVIII variants with prolonged circulatory half life.

The term "reduced capacity to bind vWF" is herein meant to encompass Factor VIII variants, wherein the capacity to bind vWF is decreased by at least 50%, preferably by at least 60%, more preferably by at least 70%, more preferably by at least 80%, more preferably by at least 90%, and most preferably about 100%. FVIII binding to vWF may be measured either by an ELISA like assay or as direct binding to immobilized vWF using surface plasmon resonance. The region in Factor VIII responsible for binding to vWF is the region spanning residues 1670-1684 as disclosed in EP0319315. It is envisaged that Factor VIII point and/or deletion mutants involving this area will modify the ability to bind to vWF. Examples of particularly preferred point mutations herein include variants comprising the following point mutations: Y1680F, Y1680R, Y1680N, Y1680C, and E1682T.

WO09156137 discloses fusion proteins with reduced vWF binding, said fusion proteins are not being conjugated with side groups such as e.g. PEG. The proteins therein are apparently useful in connection with various comparative assays.

Factor VIII molecules: FVIII/Factor VIII is a large, complex glycoprotein that primarily is produced by hepatocytes. FVIII consists of 2351 amino acids, including signal peptide, and contains several distinct domains, as defined by homology. There are three A-domains, a unique B-domain, and two C-domains. The domain order can be listed as NH2-A1-A2-B-A3-C1-C2-COOH. FVIII circulates in plasma as two chains, separated at the B-A3 border. The chains are connected by bivalent metal ion-bindings. The A1-A2-B chain is termed the heavy chain (HC) while the A3-C1-C2 is termed the light chain (LC).

Endogenous Factor VIII molecules circulate *in vivo* as a pool of molecules with B domains of various sizes. What probably occurs *in vivo* is a gradual enzymatic removal of the B domain resulting in a pool of molecules with B-domains of various sizes. It is generally believed that cleavage at position 740, by which the last part of the B-domain is removed, occurs in connection with thrombin activation. However, it cannot be ruled out that a Factor VIII variant in which e.g. the cleavage site at position 740 has been impaired may be active.

"Factor VIII" or "FVIII" as used herein refers to a human plasma glycoprotein that is a member of the intrinsic coagulation pathway and is essential to blood coagulation. "Native FVIII" is the full length human FVIII molecule as shown in SEQ ID NO. 1 (amino acid 1-2332). The B-domain is spanning amino acids 741-1648 in SEQ ID NO 1.

SEQ ID NO 1:

ATRRYYLGAVELSWDYMQSDLGELPVDARFPPRVPKS-
FPFNTSVVYKKTLFVEFTDHLFNIAKPRPPWMGLLGPTIQAEVYDTVVITLKNMASH-
PVSLHAVGVSYWKASEGAEYDDQTSQREKEDDKVFPGG-
SHTYVWQVLKENGPMASDPLCLTYSYLSH-
VDLVKDLNSGLIGALLVCREGSLAKEKTQTLHKFILLFAVF-
DEGKSWHSETKNSLMQDRDAASARAWPKMHTVNGYVNRSLPGLIGCHRKSVY-
WHVIGMGTTPEVHSIFLEGHTFLVRNHRQASLEISPITFLTAQ-
TLLMDLGQFLLFCHISSHQHDGMEAYVKVDSCPEEPQLRMKNNEEAEDYDDDLT-
DSEMDVVRFDDDNSPSFIQIRSVAKKHPKTWVHYIAAEEEDWDYAPLVLAP-
DDRSYKSQYLNNGPQRIGRKYKKVRFMAYTDETFKTREAIQHESGILGPLLYGEVGDTLLI-
IFKNQASRPYNIYPHGITDVRPLYSRRLPKGVKHLKDFPILPGEIFKYKWTVTVEDGPTKS-
DPRCLTRYYSSFVNMERDLASGLIGPLLICYKESVDQRGNQIMSDKRNVILFSVFDENRSW-
YLTENIQRFLPNPAGVQLEDPEFQASNIMHSINGYVFDSLQLSVCLHEVAYWYILSIGAQTD-
FLSVFFSGYTFKHKMVYEDTLTLFPFSGETVFMSMENPGLWILGCHNSDFRNRG-
MTALLKVSSCDKNTGDYYEDSYEDISAYLLSKNNAIEPRSFSQNSRHPSTRQKQFNATTI-
PENDIEKTDPWFAHRTPMPKIQNVSSSDLLMLLRQSPTPHGLSLSDLQEAKYETFSDDP-
SPGAIDSNNSLSEMTHFRPQLHHSGDMVFTPESGLQLRLNEKLGTTAATELKKLD-
FKVSSTSNNLISTIPSDNLAAGTDNTSSLGPPSMPVHYDSQLDTTLFGKKSSPLTESGG-
PLSLSEENNDSKLLESGLMNSQESS-
WGKNVSSTESGRLFKGKRAHGPALLTKDNALFKVSISLLKTNKTSNNSATNRKTHIDGPSLLI
ENSPSVWQNILESDTEFKKVTPLIHDRM-
LMDKNATALRLNHMSNKTTSSKNMEMVQQKKEGPIPPDAQNPDMSFFKMLFLPESAR-
WIQRTHGKNSLNSGQGP-
SPKQLVSLGPEKSVEGQNFLSEKNKVVVGKGEFTKDVGLKEMVFPSSRNLFLTNLD-
NLHENNTHNQEKKIQEEIEKKETLIQENVVLPQIHTVTGTKNFMKNLFLLSTRQNVEGSYD-
GAYAPVLQDFRSLNDSTNRTKKHTAHFSKKGEEENLEGLGNQTKQIVEKYACTTRISP-
NTSQQNFVTQRSKRALKQFRLPLEETELEKRIIVDDTSTQWSKNMKHLTPSTLTQI-
DYNEKEKGAITQSPLSDCLTRSHSIPQANRSPLPIAKVSS-
FPSIRPIYLTRVLFQDNSSHLPAASYRK-
KDSGVQESSHFLQGAKKNNLSLAILTLEMTGDQREVGSLGTSATNSVTYKKVENTVLPKPDL
PKTSGKVELLPKVHIYQKDLFPTETSNGSPGHLDLVEGSLLQGTEGAIK-
WNEANRPGKVPFLRVATESSAKTPSKLLDPLAWDNHYGTQIPKEEWKSQEKSPEKTAF-
KKKDTILSLNACESNHAIAAINEGQNKPEIEVTWAKQGRTERLCSQNPPVLKRHQREITRT-
TLQSDQEEIDYDDTISVEMKKEDFDIYDEDENQSPRSFQKKTRHYFI-

AAVERLWDYGMSSSPHVLRNRAQSGSVPQFKKVVFQEFTDGSFTQPLYRGELNEH-
LGLLGPYIRAEVEDNIMVTFRNQASRPYSFYSSLISYEEDQRQGAEPRKNFVKPNETK-
TYFWKVQHHMAPTKDEFDCKAWAYFSDVDLEKDVHSGLIG-
PLLVCHTNTLNPAHGRQVTVQEFALFFTIFDETKSWYFTENMERN-
CRAPCNIQMEDPTFKENYRFHAINGYIMDTLPGLVMAQDQRIRWYLLSMGSNE-
NIHSIHFSGHVFTVRKKEEYKMALYNLYPGVFETVEM-
LPSKAGIWRVECLIGEHLHAGMSTLFLVYSNKCQTPLGMASGHIRDFQITASGQYGQWAP-
KLARLHYSGSINAWSTKEPFSWIKVDLLAPMIIHGIKTQGARQKFSSLYISQFIIMYSLD-
GKKWQTYRGNSTGTLMVFFGNVDSSGIKHNIFNPPIIARYIRLHPTHYSIRSTLR-
MELMGCDLNSCSMPLGMESKAISDAQITASSYFTNMFAT-
WSPSKARLHLQGRSNAWRPQVNNPKEWLQVDFQKTMKVTGVTTQGVKSLLTSMYVKE-
FLISSSQDGHQWTLFFQNGKVKVFQGNQDSFTPVVNSLDPPLLTRYLRI-
HPQSWVHQIALRMEVLGCEAQDLY

SEQ ID NO 2:

ATRRYYLGAVELSWDYMQSDLGELPVDARFPPRVPKS-
FPFNTSVVYKKTLFVEFTDHLFNIAKPRPPWMGLLGPTIQAEVYDTVVITLKNMASH-
PVSLHAVGVSYWKASEGAEYDDQTSQREKEDDKVFPGG-
SHTYVWQVLKENGPMASDPLCLTYSYLSH-
VDLVKDLNSGLIGALLVCREGSLAKEKTQTLHKFILLFAVF-
DEGKSWHSETKNSLMQDRDAASARAWPKMHTVNGYVNRSLPGLIGCHRKSVY-
WHVIGMGTTPEVHSIFLEGHTFLVRNHRQASLEISPITFLTAQ-
TLLMDLGQFLLFCHISSHQHDGMEAYVKVDSCPEEPQLRMKNNEEAEDYDDDLT-
DSEMDVVRFDDDNSPSFIQIRSVAKKHPKTWVHYIAAEEEDWDYAPLVLAP-
DDRSYKSQYLNNGPQRIGRKYKKVRFMAYTDETFKTREAIQHESGILGPLLYGEVGDTLLI-
IFKNQASRPYNIYPHGITDVRPLYSRRLPKGVKHLKDFPILPGEIFKYKWTVTVEDGPTKS-
DPRCLTRYYSSFVNMERDLASGLIGPLLICYKESVDQRGNQIMSDKRNVILFSVFDENRSW-
YLTENIQRFLPNPAGVQLEDPEFQASNIMHSINGYVFDSLQLSVCLHEVAYWYILSIGAQTD-
FLSVFFSGYTFKHKMVYEDTLTLFPFSGETVFMSMENPGLWILGCHNSDFRNRG-
MTALLKVSSCDKNTGDYYEDSYEDISAYLLSKNNAIEPRSFSQNSRHP-
SQNPPVLKRHQREITRTTLQSDQEEIDYDDTISVEMKKEDFDIFDEDENQSPRSFQKK-
TRHYFIAAVERLWDYGMSSSPHVLRNRAQSGSVPQFKKVVFQEFTDGSFTQPLYR-
GELNEHLGLLGPYIRAEVEDNIMVTFRNQASRPYSFYSSLISY-
EEDQRQGAEPRKNFVKPNETKTYFWKVQHHMAPTKDEFDCKA-
WAYFSDVDLEKDVHSGLIGPLLVCHTNTLNPAHGRQVTVQEFALFFTIFDETKSWYFTEN-

MERNCRAPCNIQMEDPTFKENYRFHAINGYIMDTLPGLVMAQDQRIRWYLLSMGSNE-
NIHSIHFSGHVFTVRKKEEYKMALYNLYPGVFETVEM-
LPSKAGIWRVECLIGEHLHAGMSTLFLVYSNKCQTPLGMASGHIRDFQITASGQYGQWAP-
KLARLHYSGSINAWSTKEPFSWIKVDLLAPMIIHGIKTQGARQKFSSLYISQFIIMYSLD-
GKKWQTYRGNSTGTLMVFFGNVDSSGIKHNIFNPPIIARYIRLHPTHYSIRSTLR-
MELMGCDLNSCSMPLGMESKAISDAQITASSYFTNMFAT-
WSPSKARLHLQGRSNAWRPQVNNPKEWLQVDFQKTMKVTGVTTQGVKSLLTSMYVKE-
FLISSSQDGHQWTLFFQNGKVKVFQGNQDSFTPVVNSLDPPLLTRYLRI-
HPQSWVHQIALRMEVLGCEAQDLY

SEQ ID NO 3:

ATRRYYLGAVELSWDYMQSDLGELPVDARFPPRVPKS-
FPFNTSVVYKKTLFVEFTDHLFNIAKPRPPWMGLLGPTIQAEVYDTVVITLKNMASH-
PVSLHAVGVSYWKASEGAEYDDQTSQREKEDDKVFPGG-
SHTYVWQVLKENGPMASDPLCLTYSYLSH-
VDLVKDLNSGLIGALLVCREGSLAKEKTQTLHKFILLFAVF-
DEGKSWHSETKNSLMQDRDAASARAWPKMHTVNGYVNRSLPGLIGCHRKSVY-
WHVIGMGTTPEVHSIFLEGHTFLVRNHRQASLEISPITFLTAQ-
TLLMDLGQFLLFCHISSHQHDGMEAYVKVDSCPEEPQLRMKNNEEAEDYDDDLT-
DSEMDVVRFDDDNSPSFIQIRSVAKKHPKTWVHYIAAEEEDWDYAPLVLAP-
DDRSYKSQYLNNGPQRIGRKYKKVRFMAYTDETFKTREAIQHESGILGPLLYGEVGDTLLI-
IFKNQASRPYNIYPHGITDVRPLYSRRLPKGVKHLKDFPILPGEIFKYKWTVTVEDGPTKS-
DPRCLTRYYSSFVNMERDLASGLIGPLLICYKESVDQRGNQIMSDKRNVILFSVFDENRSW-
YLTENIQRFLPNPAGVQLEDPEFQASNIMHSINGYVFDSLQLSVCLHEVAYWYILSIGAQTD-
FLSVFFSGYTFKHKMVYEDTLTLFPFSGETVFMSMENPGLWILGCHNSDFRNRG-
MTALLKVSSCDKNTGDYYEDSYEDISAYLLSKNNAIEPRSFSQNSRHP-
SQNPPVLKRHQREITRTTLQSDQEEIDYDDTISVEMKKEDFDICDEDENQSPRSFQKK-
TRHYFIAAVERLWDYGMSSSPHVLRNRAQSGSVPQFKKVVFQEFTDGSFTQPLYR-
GELNEHLGLLGPYIRAEVEDNIMVTFRNQASRPYSFYSSLISY-
EEDQRQGAEPRKNFVKPNETKTYFWKVQHHMAPTKDEFDCKA-
WAYFSDVDLEKDVHSGLIGPLLVCHTNTLNPAHGRQVTVQEFALFFTIFDETKSWYFTEN-
MERNCRAPCNIQMEDPTFKENYRFHAINGYIMDTLPGLVMAQDQRIRWYLLSMGSNE-
NIHSIHFSGHVFTVRKKEEYKMALYNLYPGVFETVEM-
LPSKAGIWRVECLIGEHLHAGMSTLFLVYSNKCQTPLGMASGHIRDFQITASGQYGQWAP-
KLARLHYSGSINAWSTKEPFSWIKVDLLAPMIIHGIKTQGARQKFSSLYISQFIIMYSLD-

GKKWQTYRGNSTGTLMVFFGNVDSSGIKHNIFNPPIIARYIRLHPTHYSIRSTLR-
MELMGCDLNSCSMPLGMESKAISDAQITASSYFTNMFAT-
WSPSKARLHLQGRSNAWRPQVNNPKEWLQVDFQKTMKVTGVTTQGVKSLLTSMYVKE-
FLISSSQDGHQWTLFFQNGKVKVFQGNQDSFTPVVNSLDPPLLTRYLRI-
HPQSWVHQIALRMEVLGCEAQDLY

The factor VIII molecules herein may be B domain truncated Factor FVIII molecules wherein the remaining domains correspond closely to the sequence as set forth in amino acid no 1-740 and 1649-2332 in SEQ ID NO. 1 although there is of course one or more alterations within the vWF binding region between residues 1670-1684. However, B domain truncated molecules herein may differ slight from the sequence set forth in SEQ ID NO 1, meaning that the remaining domains (i.e. the three A-domains and the two C-domains) may differ slightly e.g. about 1%, 2%, 3%, 4% or 5% from the amino acid sequence as set forth in SEQ ID NO 1 (amino acids 1-740 and 1649-2332) due to the fact that mutations are introduced in order to reduce vWF binding capacity. Furthermore, it is plausible that amino acid modifications (substitutions, deletions, etc.) are introduced other places in the molecule in order to modify the binding capacity of Factor VIII with various other components such as e.g. LPR, various receptors, other coagulation factors, cell surfaces, introduction and/or abolishment of glycosylation sites, etc.

Factor VIII molecules herein have Factor VIII activity, meaning the ability to function in the coagulation cascade in a manner functionally similar or equivalent to FVIII, induce the formation of FXa via interaction with FIXa on an activated platelet, and support the formation of a blood clot. The activity can be assessed *in vitro* by techniques well known in the art such as e.g. clot analysis, endogenous thrombin potential analysis, etc. Factor VIII molecules herein have FVIII activity being at least about 10%, at least 20%, at least 30%, at least 40%, at least 50%, at least 60%, at least 70%, at least 80%, at least 90%, and 100% or even more than 100% of that of native human FVIII.

Fusion protein: Fusion proteins/ chimeric proteins, are proteins created through the joining of two or more genes which originally coded for separate proteins. Translation of this fusion gene results in a single polypeptide with functional properties derived from each of the original proteins. The fusion proteins herein comprise a FVIII polypeptide and at least one other polypeptide. The fusion protein can optionally comprise at least one linker. Thus, the FVIII polypeptide may not be directly linked to the other polypeptide moiety. The other polypeptide moiety can be joined to the N- or C-terminus of a FVIII polypeptide chain or inserted at an internal position of a FVIII polypeptide chain.

In a preferred embodiment, the other polypeptide moiety is inserted into the B domain linker of B domain-deleted FVIII. In another preferred embodiment, the other polypeptide moiety replaces the vWF-binding a3 region at the N-terminus of the FVIII light chain. In another preferred embodiment, the other polypeptide moiety is joined to C-terminus of the FVIII light chain.

The other polypeptide may comprise one or more amino acid sequences derived from e.g. human serum albumin, an antibody binding polypeptide, an Fc receptor, human FcγRI (CD64), human chorion gonadotropin, the Fc portion of an antibody (immunoglobulin), transferring, an albumin binding polypeptide, or a transferrin binding polypeptide.

B domain: The B-domain in Factor VIII spans amino acids 741-1648 in SEQ ID NO 1. The B-domain is cleaved at several different sites, generating large heterogeneity in circulating plasma FVIII molecules. The exact function of the heavily glycosylated B-domain is unknown. What is known is that the domain is dispensable for FVIII activity in the coagulation cascade. This apparent lack of function is supported by the fact that B domain deleted/truncated FVII appears to have *in vivo* properties identical to those seen for full length native FVIII. That being said there are indications that the B-domain may reduce the association with the cell membrane, at least under serum free conditions.

B domain truncated/deleted Factor VIII molecule: Endogenous full length FVIII is synthesized as a single-chain precursor molecule. Prior to secretion, the precursor is cleaved into the heavy chain and the light chain. Recombinant B domain-deleted FVIII can be produced from two different strategies. Either the heavy chain without the B-domain and the light chain are synthesized individually as two different polypeptide chains (two-chain strategy) or the B-domain deleted FVIII is synthesized as a single precursor polypeptide chain (single-chain strategy) that is cleaved into the heavy and light chains in the same way as the full-length FVIII precursor.

In a B domain-deleted FVIII precursor polypeptide, the heavy and light chain moieties are normally separated by a linker. To minimize the risk of introducing immunogenic epitopes in the B domain-deleted FVIII, the sequence of the linker is preferable derived from the FVIII B-domain. As a minimum, the linker must comprise a recognition site for the protease that separates the B domain-deleted FVIII precursor polypeptide into the heavy and light chain. In the B domain of full length FVIII, amino acid 1644-1648 constitutes this recognition site. The thrombin site leading to removal of the linker on activation of B domain-deleted FVIII is located in the heavy chain. Thus, the size and amino acid sequence of the linker is unlikely to influence its removal from the remaining FVIII molecule by thrombin activation. Deletion of the B domain is an advantage for production of FVIII. Nevertheless, parts of the B domain can be included in the linker without reducing the productivity. The negative effect of the B domain on productivity has not been attributed to any specific size or sequence of the B domain.

The truncated B-domain may contain several O-glycosylation sites. However, according to a preferred embodiment, the molecule comprises only one, alternatively two, three or four O-linked oligosaccharides in the truncated B-domain.

According to a preferred embodiment, the truncated B domain comprises only one potential O-glycosylation sites and a hydrophilic polymer is covalently conjugated to this O-glycosylation site.

The O-linked oligosaccharides in the B-domain truncated molecules herein may be attached to O-glycosylation sites that were either artificially created by recombinant means and/or by exposure of "hidden" O-glycosylation sites by truncation of the B-domain. In both cases, such molecules may be made by designing a B-domain trunctated Factor VIII amino acid sequence and subsequently subjecting the amino acid sequence to an *in silico* analysis predicting the probability of O-glycosylation sites in the truncated B-domain. Molecules with a relatively high probability of having such glycosylation sites can be synthesized in a suitable host cell followed by analysis of the glycosylation pattern and subsequent selection of molecules having O-linked glycosylation in the truncated B-domain.

The Factor VIII molecule also contains a number of N-linked oligosaccharides that may also serve as conjugation points using either enzymatic or chemical conjugation methods, e.g., sialyltransferases combined with sialic acid derivatives or glycans oxidation to form aldehydes for subsequent modification by select reagents.

Suitable host cells for producing recombinant factor VIII protein are preferably of mammalian origin in order to ensure that the molecule is glycosylated. The cells are preferably mammalian cells, more preferably an established mammalian cell line, including, without limitation, CHO (e.g., ATCC CCL 61), COS-1 (e.g., ATCC CRL 1650), baby hamster kidney (BHK), and HEK293 (e.g., ATCC CRL 1573; Graham et al., J. Gen. Virol. 36:59-72, 1977) cell lines. A preferred BHK cell line is the tk- ts13 BHK cell line (Waechter and Baserga, Proc.Natl.Acad.Sci.USA 79:1106-1110, 1982), hereinafter referred to as BHK 570 cells. The BHK 570 cell line is available from the American Type Culture Collection, 12301 Parklawn Dr., Rockville, MD 20852, under ATCC accession number CRL 10314. A tk- ts13 BHK cell line is also available from the ATCC under accession number CRL 1632. A preferred CHO cell line is the CHO K1 cell line available from ATCC under accession number CCI61 as well as cell lines CHO-DXB11 and CHO-DG44.

Other suitable cell lines include, without limitation, Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1); DUKX cells (CHO cell line) (Urlaub and Chasin, Proc. Natl. Acad. Sci. USA 77:4216-4220, 1980) (DUKX cells also being referred to as DXB11 cells), and DG44 (CHO cell line) (Cell, 33: 405, 1983, and Somatic Cell and Molecular Genetics 12: 555, 1986). Also useful are 3T3 cells, Namalwa cells, myelomas and fusions of myelomas with other cells. In some embodiments, the cells may be mutant or recombinant cells, such as, e.g., cells that express a qualitatively or quantitatively different spectrum of enzymes that catalyze post-translational modification of proteins (e.g., glycosylation enzymes such as glycosyl transferases and/or glycosidases, or processing enzymes such as propeptides) than the cell type from which they were derived. DUKX cells (CHO cell line) are especially preferred.

Currently preferred cells are HEK293, COS, Chinese Hamster Ovary (CHO) cells, Baby Hamster Kidney (BHK) and myeloma cells, in particular Chinese Hamster Ovary (CHO) cells.

It thus appears that it is possible to active "hidden" O-glycosylation sites in the factor VIII B-domain by truncating the B-domain. While not wishing to be bound by any theory, this phenomenon could be attributable to the tertiary structure of the molecule in the truncated B-domain being altered. "Hidden" O-glycosylation sites are thus "made accessible" to glycosylation in the truncated B-domain. One advantage of this approach is the provision of recombinant molecules with an advantageous safety profile with respect to e.g. allergenicity. Another advantage could be that it may represent a simpler approach of obtaining B-domain truncated variants with an O-linked oligosaccharide in the B-domain due to the inherent abundance of glycosylation sites in the B-domain as it has previously proven difficult to engineer artificial O-glycosylation sites in recombinant proteins.

The length of the B domain in the wt FVIII molecule is about 907 amino acids. The length of the truncated B domain in molecules herein may vary from about 10 to about 800 amino acids, such as e.g. from about 10 amino acids to about 700 acids, such as e.g. about 12-500 amino acids, 12-400 amino acids, 12-300 amino acids, 12-200 amino acids, 15-100 amino acids, 15-75 amino acids, 15-50 amino acids, 15-45 amino acids, 20-45 amino acids, 20-40 amino acids, or 20-30 amino acids. The truncated B-domain may comprise fragments of the heavy chain and/or the light chain and/or an artificially introduced sequence that is not found in the wt FVIII molecule. The terms "B-domain truncated" and "B-domain deleted" may be used interchangeably herein.

Modified circulatory half life: Molecules herein have a modified circulatory half life compared to the wild type Factor VIII molecule, preferably an increased circulatory half life. Circulatory half life is preferably increased at least 10%, preferably at least 15%, preferably at least 20%, preferably at least 25%, preferably at least 30%, preferably at least 35%, preferably at least 40%, preferably at least 45%, preferably at least 50%, preferably at least 55%, preferably at least 60%, preferably at least 65%, preferably at least 70%, preferably at least 75%, preferably at least 80%, preferably at least 85%, preferably at least 90%, preferably at least 95%, preferably at least 100%, more preferably at least 125%, more preferably at least 150%, more preferably at least 175%, more preferably at least 200%, and most preferably at least 250% or 300%. Even more preferably, such molecules have a circulatory half life that is increased at least 400%, 500%, 600%, or even 700%.

Side chain/side group: FVIII variants herein may be covalently conjugated with a side group either via post-translational modification or in the form of a fusion protein. One or more of the following side group modifications of FVIII may thus be carried out: alkylation, acylation, ester formation, di-sulfide or amide formation or the like. This includes PEGylated FVIII, cysteine-PEGylated FVIII and variants thereof. The FVIII variants herein may also be conjugated to biocompatible fatty acids and derivatives thereof, hydrophilic polymers (Hydroxy Ethyl Starch, Poly Ethylen Glycol, hyaluronic acid, Phosphorylcholine-based polymers, fleximers, dextran, poly-sialic acids), polypeptides (antibodies, antigen binding fragments of antibodies, Fc domains, transferrin, albumin, Elastin like peptides (MacEwan SR, Chilkoti A. Biopolymers. 2010;94:60), XTEN polymers (Schellenberger V et al. Nat Biotechnol. 2009;27:1186), PASylation or HAPylation (Schlapschy M et al. Protein Eng Des Sel. 2007 ;20 :273), Albumin binding peptides (Dennis MS et al. J Biol Chem. 2002, 277:35035)), etc.

FVIII molecules herein may be conjugated by one or more hydrophobic side groups - optionally via a linker. Compounds having a -(CH₂)₁₂-moiety are possible albumin binders in the context of the present invention. Hydrophobic side groups may sometimes be referred to as "albumin binders" due to the fact that such side groups may be capable of forming non-covalent complexes with albumin, thereby promoting the circulation of the modified FVIII variant in the blood stream, due to the fact that the complexes of the modified FVIII variant and albumin is only slowly disintegrated to release the FVIII variant. FVIII can be modified using chemical methods as well as enzymatic "glyco-modification" methods essentially following the processes as disclosed in WO03031464. Enzymatic methods have the advantages of avoiding use of any organic solvents as well as being very site specific in general.

The term "PEGylated FVIII" means FVIII, conjugated with a PEG molecule. It is to be understood, that the PEG molecule may be attached to any part of FVIII including any amino acid residue or carbohydrate moiety. The term "cysteine-PEGylated FVIII" means FVIII having a PEG molecule conjugated to a sulfhydryl group of a cysteine introduced in FVIII.

PEG is a suitable polymer molecule, since it has only few reactive groups capable of cross-linking compared to polysaccharides such as dextran. In particular, monofunctional PEG, e.g. methoxypolyethylene glycol (mPEG), is of interest since its coupling chemistry is relatively simple (only one reactive group is available for conjugating with attachment groups on the polypeptide). Consequently, the risk of cross-linking is eliminated, the resulting polypeptide conjugates are more homogeneous and the reaction of the polymer molecules with the polypeptide is easier to control.

To effect covalent attachment of the polymer molecule(s) to the polypeptide, the hydroxyl end groups of the polymer molecule are provided in activated form, i.e. with reactive functional groups. The PEGylation may be directed towards conjugation to all available attachment groups on the polypeptide (i.e. such attachment groups that are exposed at the surface of the polypeptide) or may be directed towards one or more specific attachment groups, e.g. the N-terminal amino group (U.S. Pat. No. 5,985,265), N- and/or O-linked glycans, etc. Furthermore, the conjugation may be achieved in one step or in a stepwise manner (e.g. as described in WO 99/55377). An enzymatic approach for coupling side groups to O- and/or N-linked glycans is disclosed in WO03031464.

Fusion protein: Fusion proteins/chimeric proteins, are proteins created through the joining of two or more genes which originally coded for separate proteins. Translation of this fusion gene results in a single polypeptide with functional properties derived from each of the original proteins. The side chain of the FVIII variants herein may thus be in the form of a polypeptide fused to FVIII. FVIII variants herein may thus be fused to peptides that can confer a prolonged half life to the FVIII such as e.g. antibodies and "Fc fusion derivatives" or "Fc fusion proteins".

Fc fusion protein is herein meant to encompass FVIII fused to an Fc domain that can be derived from any antibody isotype, although an IgG Fc domain will often be preferred due to the relatively long circulatory half life of IgG antibodies. The Fc domain may furthermore be modified in order to modulate certain effector functions such as e.g. complement binding and/or binding to certain Fc receptors. Fusion of FVIII with an Fc domain, having the capacity to bind to FcRn receptors, will generally result in a prolonged circulatory half life of the fusion protein compared to the half life of the wt FVIII protein. Mutations in positions 234, 235 and 237 in an IgG Fc domain will generally result in reduced binding to the FcγRI receptor and possibly also the FcγRIIa and the FcγRIII receptors. These mutations do not alter binding to the FcRn receptor, which promotes a long circulatory half life by an endocytic recycling pathway. Preferably, a modified IgG Fc domain of a fusion protein herein comprises one or more of the following mutations that will result in decreased affinity to certain Fc receptors (L234A, L235E, and G237A) and in reduced C1q-mediated complement fixation (A330S and P331S), respectively.

Without being bound by theory it is envisaged that the reason why it functions more efficiently to attach side groups to Factor VIII molecules with reduced vWF binding capacity rather than attaching side groups to Factor VIII molecules with normal vWF binding capacity is that the relative size of the side group is relatively small in the large Factor VIII/vWF complex. It is hypothesized that a relatively large side group functions more efficiently in shielding the free Factor VIII from clearance. It is further hypothesized that the half life of FVIII is related to that of vWF. FVIII molecules with reduced ability to bind vWF most likely have exposed clerance epitopes which would normally have been shielded by vWF. By attaching side groups it is thus hypothesized that this "clearance shielding" can be regained. In other cases, attachment of side groups such as e.g. antibody fragments may function by e.g. attaching the molecule to proteins, cells, or platelets having a relatively long circulatory half life.

Hydrophilic polymer: The modifying group/hydrophilic polymer herein is preferably non-naturally occurring. In one example, the "non-naturally occurring modifying group" is a polymeric modifying group, in which at least one polymeric moiety is non-naturally occurring. In another example, the non-naturally occurring modifying group is a modified carbohydrate. The locus of functionalization with the modifying group is selected such that it does not prevent the "modified sugar" from being added enzymatically to a polypeptide. "Modified sugar" also refers to any glycosyl mimetic moiety that is functionalized with a modifying group and which is a substrate for a natural or modified enzyme, such as a glycosyltransferase.

The polymeric modifying group added to a polypeptide can alter a property of such polypeptide, for example, its bioavailability, biological activity or its half-life in the body. Exemplary polymers herein include water soluble polymers that can be linear or branched and can include one or more independently selected polymeric moieties, such as poly(alkylene glycol) and derivatives thereof. The polymeric modifying group herein may include a water-soluble polymer, e.g. poly(ethylene glycol) and derivatived thereof (PEG, m-PEG), poly(propylene glycol) and derivatives thereof (PPG, m-PPG) and the like.

The term "water-soluble" refers to moieties that have some detectable degree of solubility in water. Methods to detect and/or quantify water solubility are well known in the art. Exemplary water-soluble polymers herein include peptides, saccharides, poly(ethers), poly(amines), poly(carboxylic acids) and the like. Peptides can have mixed sequences and be composed of a single amino acid, e.g., poly(lysine). An exemplary polysaccharide is poly(sialic acid). An exemplary poly(ether) is poly(ethylene glycol), e.g., m-PEG. Poly(ethylene imine) is an exemplary polyamine, and poly(acrylic) acid is a representative poly(carboxylic acid).

The polymer backbone of the water-soluble polymer herein can be poly(ethylene glycol) (i.e. PEG). The term PEG includes poly(ethylene glycol) in any of its forms, including alkoxy PEG, difunctional PEG, multiarmed PEG, forked PEG, branched PEG, pendent PEG (i.e. PEG or related polymers having one or more functional groups pendent to the polymer backbone), or PEG with degradable linkages therein.

The polymer backbone can be linear or branched. Branched polymer backbones are generally known in the art. Typically, a branched polymer has a central branch core moiety and a plurality of linear polymer chains linked to the central branch core. PEG is commonly used in branched forms that can be prepared by addition of ethylene oxide to various polyols, such as glycerol, pentaerythritol and sorbitol. The central branch moiety can also be derived from several amino acids, such as lysine or cysteine. In one example, the branched poly(ethylene glycol) can be represented in general form as R(-PEG-OH)m in which R represents the core moiety, such as glycerol or pentaerythritol, and m represents the number of arms. Multi-armed PEG molecules, such as those described in U.S. Patent No. 5,932,462, which is incorporated by reference herein in its entirety, can also be used as the polymer backbone. Many other polymers are also suitable herein. Polymer backbones that are non-peptidic and water-soluble, are particularly useful. Examples of suitable polymers include, but are not limited to, other poly(alkylene glycols), such as poly(propylene glycol) ("PPG"), copolymers of ethylene glycol and propylene glycol and the like, poly(oxyethylated polyol), poly(olefmic alcohol), poly(vinylpyrrolidone), poly(hydroxypropylmethacrylamide), poly([alpha] -hydroxy acid), poly(vinyl alcohol), polyphosphazene, polyoxazoline, poly(N-acryloylmorpholine), such as described in U.S. Patent No. 5,629,384, which is incorporated by reference herein in its entirety, as well as copolymers, terpolymers, and mixtures thereof.

Although the molecular weight of each chain of the polymer backbone can vary, it is typically in the range of from about 100 Da to about 160,000 Da, such as e.g. from about 5,000 Da to about 100,000 Da. More specifically, the size of each conjugated hydrophilic polymer herein may vary from about 500 Da to about 80,000 Da, such as e.g. about 1000 Da to about 80,000 Da; about 2000 Da to about 70,000 Da; about 5000 to about 70,000 Da; about 5000 to about 60,000 Da; about 10,000 to about 70,000 Da; about 20,000 to about 60,000 Da; about 30,000 to about 60,000 Da; about 30,000 to about 50,000 Da; or about 30,000 to about 40,000 Da. It should be understood that these sizes represent estimates rather than exact measures. The molecules herein can be conjugated with a heterogenous population of hydrophilic polymers, such as e.g. PEG of a size of e.g. 10,000, 40,000, or 80,000 Da +/- about 5000, about 4000, about 3000, about 2000, or about 1000 Da.

### Albumin binder conjugates/side groups

It is known that the *in vivo* properties of proteins can be improved by the use of albumin binding side chains. Such side chains, or albumin binders, can be attached to the protein prior to administration and can, for example, stabilise the protein *in vivo* or improve or extend the *in vivo* half-life of the protein.

The albumin binder may thereby promote the circulation of the derivative with the blood stream. The albumin binder may have the effect of extending or protracting the time of action of the protein that it is bound to it, due to the fact that the complexes of the peptide derivative and albumin are only slowly disintegrated to release the active pharmaceutical ingredient. Thus, a preferred substituent, or side chain, as a whole may be referred to as an albumin binding moiety.

The albumin binder (albumin binding moiety) may comprise a portion which is particularly relevant for the albumin binding and thereby the protraction of circulation in the blood stream, which portion may accordingly be referred to as a protracting moiety. The protracting moiety is preferably at, or near, the opposite end of the albumin binding moiety as compared to its point of attachment to the peptide.

In a preferred embodiment, the albumin binder is, or comprises, a side chain that is capable of forming non-covalent complexes with albumin. The albumin binder may bind albumin non-covalently and/or reversibly. The albumin binder may bind albumin specifically. As is clear from the methods described below, the albumin binder may bind to cyclodextrin. The albumin binder may bind cyclodextrin non-covalently and/or reversibly. The albumin binder may bind cyclodextrin specifically.

An albumin binder as described herein is generally a hydrophobic group.

The other portion of the albumin binding moiety, i.e. the portion in-between the protracting moiety and the point of attachment to the peptide, may be referred to as a linker moiety, linker, spacer, or the like. However, the presence of such a linker is optional, and hence the albumin binding moiety may be identical to the protracting moiety.

In particular embodiments, the albumin binding moiety and/or the protracting moiety is lipophilic, and/or negatively charged at physiological pH (7.4).

The albumin binding moiety and/or the protracting moiety may be covalently attached to an amino group of the peptide by conjugation chemistry such as by alkylation, acylation, or amide formation; or to a hydroxyl group, such as by esterification, alkylation, oximation.

In a preferred embodiment, an active ester of the albumin binding moiety and/or the protracting moiety is covalently linked to an amino group of a sialic acid residue or a sialic acid derivative, under formation of an amide bond.

For the present purposes, the terms "albumin binding moiety", "protracting moiety", and "linker" include the un-reacted as well as the reacted forms of these molecules. Whether or not one or the other form is meant is clear from the context in which the term is used.

The albumin binding moiety may be, or may comprise a fatty acid or fatty diacid or a derivative or either thereof.

The term "fatty acid" refers to aliphatic monocarboxylic acids having from 4 to 28 carbon atoms, such as 16 carbon atoms. It is preferably unbranched, and/or even numbered, and it may be saturated or unsaturated.

The term "fatty diacid" refers to fatty acids as defined above but with an additional carboxylic acid group in the omega position. Thus, fatty diacids are dicarboxylic acids.

The nomenclature is as is usual in the art, for example -COOH, as well as HOOC-, refers to carboxy; -C₆H₄- to phenylen; -CO-, as well as -OC-, to carbonyl (O=C<); and C₆H₅-O- to phenoxy.

In a preferred embodiment the linker moiety, if present, has from 2 to 80 C-atoms, preferably from 5 to 70 C-atoms. In additional preferred embodiments, the linker moiety, if present, has from 4 to 20 hetero atoms, preferably from 2 to 40 hetero atoms, more preferably from 3 to 30 hetero atoms. Particularly preferred examples of hetero atoms are N-, and O-atoms. H-atoms are not hetero atoms.

In another embodiment, the linker comprises at least one OEG molecule, and/or at least one glutamic acid residue, or rather the corresponding radicals (OEG designates 8-amino-3,6-dioxaoctanic acid, i.e. this radical: -NH-(CH₂)₂-O-(CH₂)₂-O-CH₂-CO-).

In one preferred embodiment, the linker moiety comprises a di-carboxyl residue linked to a sialic acid residue by an amide bond. In preferred examples, the di-carboxyl residue has from 2-30 C-atoms, preferably 4-20 C-atoms, more preferably 4-10 C-atoms. In additional preferred examples, the di-carboxyl residue has from 0-10 hetero-atoms, preferably 0- 5 hetero-atoms.

In another preferred example, the linker moiety comprises a group containing both an amino and a distal carboxyl-group linked to a sialic acid residue by an amide bond through its distal carboxyl groups. In one preferred embodiment the this group is an OEG group.

The amino acid glutamic acid (Glu) comprises two carboxylic acid groups. Its gamma-carboxy group is preferably used for forming an amide bond with an amino group of a sialic acid residue or a sialic acid derivative, or with an amino group of an OEG molecule, if present, or with the amino group of another Glu residue, if present. The amino group of Glu in turn forms an amide bond with the carboxy group of the protracting moiety, or with the carboxy group of an OEG molecule, if present, or with the gamma-carboxy group of another Glu, if present. This way of inclusion of Glu is occasionally briefly referred to as "gamma-Glu".

O-linked oligosaccharide: Both N-glycans and O-glycans are attached to proteins by the cells producing the protein. The cellular N-glycosylation machinery recognizes and glycosylates N-glycosylation signals (N-X-S/T motifs) in the amino acid chain, as the nascent protein is translocated from the ribosome to the endoplasmic reticulum (Kiely et al. 1976; Glabe et al. 1980).

Likewise, O-glycans are attached to specific O-glycosylation sites in the amino acid chain, but the motifs triggering O-glycosylation are much more heterogenous than the N-glycosylation signals, and our ability to predict O-glycosylation sites in amino acid sequences is still inadequate (Julenius et al. 2004). The construction of artificial O-glycosylation sites is thus associated with some uncertainty.

An O-linked oligosaccharide in a truncated Factor VIII B domain may thus be covalently linked to a naturally occurring O-linked glycosylation sequence or an O-linked glycosylation sequence which has been artificially constructed by recombinant techniques.

Preferably, an O-linked oligosaccharide is linked to a naturally occurring O-linked glycosylation sequence which is not exposed to glycosylation in the wild type Factor VIII molecules but is becoming accessible to O-glycosylation as a consequence of truncation of the B domain. An example thereof is a B-domain truncated Factor VIII variant wherein the B-domain corresponds to amino acids 742-763 in SEQ ID NO1. It is plausible that the "hidden" O-glycosylation site in this truncated variant will also become glycosylated even if the B-domain is truncated at a somewhat different place, i.e. if the truncated B domain is somewhat shorter (e.g. 1, 2, 3, 4, or 5 amino acids shorter) or longer (such as e.g. 1, 2, 3, 4, 5, 6, 7, 8, 9, 10, 15, 20, 25, 30, 35, 40, 45, or 50 amino acids) compared to the 742-763 linker. This approach by activating a "hidden" O-glycosylation site by truncation of a B-domain rather than creation of an artificial O-glycosylation site has the advantage of creating a molecule with an advantageous safety profile (i.e. reduced allergenicity, etc.). Other O-glycosylation sites in the Factor VIII B-domain may likewise become activated by truncating the molecules in different ways.

Sialyltransferase: Sialyltransferases are enzymes that transfer sialic acid to nascent oligosaccharide. Each sialyltransferase is specific for a particular sugar substrate. Sialyltransferases add sialic acid to the terminal portions of the sialylated glycolipids (gangliosides) or to the N- or O-linked sugar chains of glycoproteins. There are about twenty different sialyltransferases which can be distinguished on the basis of the acceptor structure on which they act and on the type of sugar linkage they form. Preferred sialyltransferases herein are ST3Gal-I (specific for O-glycans) and ST3Gal-III (specific for N-glycans). It is thus possible to engineer the structure of the conjugated Factor VIII molecules herein by e.g. selection of a specific sialyltransferase and/or engineering of a Factor VIII molecule with a particular glycosylation pattern.

Glyco-PEGylation of O-linked oligosaccharide: The biosynthesis of O-glycans can be modified and terminated with the addition of sialic acid residues relatively early in biosynthesis. Certain sialyltransferase enzymes are capable of acting on GalNAcα-Ser/Thr, or early O-glycan core subtypes after Core 1 GalT action. The term T antigenis associated with the presence of the Galβ1-3GalNAcα-Ser/Thr disaccharide. Production of these structures involves a competition among glycosyltransferases for the same substrate and thus thue expression levels and subcellular distributions of glycosyltransferases within the Golgi apparatus determines the structural outcome in O-glycan biosynthesis and diversification. Only the Galβ1-3GalNAcα-Ser/Thr disaccharide is amenable for glycoPEGylation.

However, the available amount of this structure may be greatly enhanced through treatment of the protein with sialidase or Core1 GalT or a combination thereof. As a result of the glycoPEGylation process the Sialic acid PEG is added to the native structure through an α3 bond to the Galβ1-3GalNAcα-Ser/Thr disaccharide of the target protein.

Other hydrophilic polymers can also be attached to O-linked oligosaccharides. The basic requirement for enzymatically conjugating other hydrophilic polymers to FVIII via the O-glycan is the ability to couple them to the glycyl-Sialic acid derivative via the free amino group as disclosed in WO03031464. This may be achieved through a large variety of coupling chemistries known to those skilled in the art. Examples of activated biocompatible polymer includes polyalkylene oxides such as without limitation polyethylene glycol (PEG), 2-(methacryloyloxy)ethyl phosphorylcholine (mPC) polymers (as described in WO03062290), dextrans, colominic acids or other carbohydrate based polymers, polymers of amino acids or of specific peptides sequences, biotin derivatives, polyvinyl alcohol (PVA), polycarboxylates, polyvinylpyrrolidone, polyethylene-co-maleic acid anhydride, polystyrene-co-malic acid anhydride, polyoxazoline, poly-acryloylmorpholine, heparin, albumin, celluloses, hydrolysates of chitosan, starches such as hydroxyethyl-starches and hydroxy propyl-starches, glycogen, agaroses and derivatives thereof, guar gum, pullulan, inulin, xanthan gum, carrageenan, pectin, alginic acid hydrolysates, other bio-polymers and any equivalents thereof.

Side groups can be attached to N-linked oligosaccharides as disclosed in e.g. WO0331464. Such methods frequently result in attachment of several side groups to the Factor VIII molecule.

Pharmaceutical composition: A pharmaceutical composition is herein preferably meant to encompass compositions comprising Factor VIIII molecules herein suitable for parenteral administration, such as e.g. ready-to-use sterile aqueous compositions or dry sterile compositions that can be reconstituted in e.g. water or an aqueous buffer. The compositions herein may comprise various pharmaceutically acceptable excipients, stabilizers, etc.

Additional ingredients in such compositions may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation. Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of the FVIII compound in the form of a nasal or pulmonal spray. As a still further option, the pharmaceutical compositions containing the FVIII compound herein may also be adapted to transdermal administration, e.g. by needle-free injection or from a patch, optionally an iontophoretic patch, or transmucosal, e.g. buccal, administration.

The term "treatment", as used herein, refers to the medical therapy of any human or other animal subject in need thereof. Said subject is expected to have undergone physical examination by a medical practitioner, who has given a tentative or definitive diagnosis which would indicate that the use of said specific treatment is beneficial to the health of said human or other animal subject. The timing and purpose of said treatment may vary from one individual to another, according to the status quo of the subject's health. Thus, said treatment may be prophylactic, palliative, symptomatic and/or curative.

In a first aspect the present invention thus relates to a recombinant Factor VIII molecule, wherein said molecule has prolonged circulatory half-life and reduced vWF binding capacity, and wherein said molecule is covalently conjugated with at least one side group such that said side group is selected from one or more of the group consisting of: hydrophilic polymer, an albumin binder, an antibody or a fragment thereof, and albumin; and wherein the molecule comprises a point mutation and/or a deletion in the region spanning amino acid residues 1670-1684 in SEQ ID NO: 1.

In a preferred embodiment, the Factor VIII molecule is a B-domain truncated variant and the side group is optionally covalently conjugated to the truncated B-domain, whereby Factor VIII activation results in removal of the covalently conjugated side group. In another embodiment, the B-domain truncated molecule is covalently conjugated with a hydrophilic polymer via an O-linked oligosaccharide in the truncated B domain, and wherein Factor VIII activation results in removal of the covalently conjugated hydrophilic polymer. Such an O-glycosylation site is preferably constructed by truncation of the B-domain.b

In yet another preferred embodiment, the molecule according to the invention comprises a point mutation in residue 1680. It follows that molecules herein may comprise both point mutations and deletions within the vWF binding domain. According to a particularly preferred embodiment, the side group is PEG.

Another aspect of the present invention relates to a method of making a molecule according to the invention, wherein said method comprises attachment of a side group to a Factor VIII molecule having reduced capacity to bind vWF. Molecules obtainable by or obtained by such methods are also described herein. A third aspect of the present invention relates to a method of treatment of a haemophilic disease comprising administering to a patient in need thereof a therapeutically effective amount of a molecule according to the invention.

A fourth aspect of the invention relates to use of a molecule according to the invention as a medicament.

A fifth aspect relates to use of a molecule according to the invention for manufacture of a medicament for treatment of haemophilia.

A final aspect relates to a pharmaceutical composition comprising a molecule according to the invention.

### EXAMPLES

### Example 1 Production of recombinant B domain truncated O-glycosylated Factor VIII and varints thereof, e.g., Factor VIII (Y1680F) or Factor VIII (Y1680C)

### Cell line and culture process

Using Factor VIII cDNA, a mammalian expression plasmid was constructed. The plasmids encodes a B-domain deleted Factor VIII comprising the Y1680F mutation, the Factor VIII heavy chain comprising amino acid 1-740 of full length human Factor VIII, and Factor VIII light chain comprising amino acid 1649-2332 of full length human Factor VIII. The heavy and light chain sequences are connected by a 21 amino acid linker (SFSQNSRHP-SQNPPVLKRHQR - SEQ ID NO 4) comprising the sequence of amino acid 741-750 and 1638-1648 of full length human Factor VIII. FVIII variants comprising thie linker defined in SEQ ID NO 4 may alo herein be referred to as "N8". The Factor VIII amino acid sequence encoded by this plasmid is as set forth in **SEQ ID NO 1 (wt), SEQ ID NO 2 (Y1680F), SEQ ID NO 3 (Y1680C)**

Chinese hamster ovary (CHO) cells were transfected with the plasmid and selected with the dihydrofolate reductase system eventually leading to a clonal suspension producer cell cultivated in animal component-free medium.

The first step in the process is the inoculation of a cell vial, from a working cell bank vial, into a chemically defined and animal component free growth medium. Initially after thawing, the cells are incubated in a T-flask. One or two days after thawing, the cells are transferred to a shaker flask, and the culture volume is expanded by successive dilutions in order to keep the cell density between 0.2 - 3.0 x 10⁶ cells/ml. The next step is the transfer of the shaker flask culture into seed bioreactors. The culture volume is here further expanded before the final transfer to the production bioreactor. The same chemically defined and animal component free medium is used for all the inoculum expansion steps. After transfer to the production bioreactor, the medium is supplemented with components that increase the product concentration. In the production bioreactor the cells are cultured in a repeated batch process with a cycle time of three days. At harvest, 80 - 90 % of the culture volume is transferred to a harvest tank. The remaining culture fluid is then diluted with fresh medium, in order to obtain the initial cell density, and then a new growth period is initiated. The harvest batch is clarified by centrifugation and filtration and transferred to a holding tank before initiation of the purification process. A buffer is added to the cell free harvest in the holding tank to stabilise pH.

By the end of the production run, cells are collected and frozen, in order to make an end of production cell bank. This cell bank is tested for mycoplasma, sterility and viral contamination.

### Purification

For the isolation of B-domain-deleted Factor VIII (Y1680F) from cell culture media a four step purification procedure was used including a concentration step on a Capto MMC column, an immunoabsorbent chromatography step, an anionic exchange chromatography and finally a gelfiltration step. Typically the following procedure was used: 11 litre of sterile filtered medium was pumped onto at column (1.6 x 12 cm) of Capto MMC (GE Healthcare, Sweden) equilibrated in buffer A: 20 mM imidazole, 10 mM CaCl₂, 50 mM NaCl, 0.02 % Tween 80, pH = 7.5 at a flow of 15 ml/min. The column was washed with 75 ml of buffer A followed by wash with 75 ml of buffer A containing 1.5 M NaCl. The protein was eluted with 20 mM imidazole, 10 mM CaCl₂, 0.02 % Tween 80, 2.5 M NaCl, 8 M ethyleneglycol, pH = 7.5 at a flow of 1 ml/min. Fractions of 8 ml were collected and assayed for Factor VIII activity (FVIII:C) in a chromogenic assay (see example 3). Factor VIII containing fractions were pooled and normally a pool volume of around 50 ml was obtained.

A monoclonal antibody against Factor VIII has been developed (Kjalke Eur J Biochem 234 773, Hansen, J Thromb Haemost 2009; 7, Supplement 2: abstract no. PP-WE-572). By further epitope mapping (results not shown) this antibody, F25, was found to recognise the far C-terminal sequence of the heavy chain from amino acid residue 725 to 740. The F25 antibody was coupled to NHS-activated Sepharose 4 FF (GE Healthcare, Bio-Sciences AB, Uppsala, Sweden) at a density of 2.4 mg per ml of gel essentially as described by the manufacturer. The pool from the previous step was diluted 10 times with 20 mM imidazole, 10 mM CaCl₂, 0.02 % Tween 80, pH = 7.3 and applied to the F25 Sepharose column (1.6 x 9.5 cm) equilibrated with 20 mM imidazole, 10 mM CaCl₂, 150 mM NaCl, 0.02 % Tween 80, 1 M glycerol pH = 7.3 at a flow of 0.5 ml/min. The column was washed with equilibration buffer until the UV signal was constant and then with 20 mM imidazole, 10 mM CaCl₂, 0.65 M NaCl, pH = 7.3 until the UV signal was constant again. Factor VIII was eluted with 20 mM imidazole, 10 mM CaCl₂, 0.02 % Tween 80, 2.5 M NaCl, 50% ethyleneglycol, pH = 7.3 at a flow of 1 ml/min. Fractions of 1 ml were collected and assayed for Factor VIII:C (see example 3). Factor VIII containing fractions were pooled and normally a pool volume of around 25 ml was obtained.

A buffer A: 20 mM imidazole, 10 mM CaCl₂, 0.02 % Tween 80, 1 M glycerol, pH = 7.3 and a buffer B: 20 mM imidazole, 10 mM CaCl₂, 0.02 % Tween 80, 1 M glycerol, 1 M NaCl, pH = 7.3 was prepared for the ion-exchange step. A column (1 x 10 cm) of Macro-Prep 25Q Support (Bio-Rad Laboratories, Hercules, CA, USA) was equilibrated with 85% buffer A/15% Buffer B at a flow of 2 ml/min. The pool from the previous step was diluted 10 times with buffer A and pumped onto the column with a flow of 2 ml/min. The column was washed with 85% buffer A/15% buffer B at a flow of 2 ml/min and Factor VIII was eluted with a linear gradient from 15 % buffer B to 70 % buffer B over 120 ml at a flow of 2 ml/min. Fractions of 2 ml were collected and assayed for Factor VIII activity (FVIII:C) as described in example 3. Factor VIII containing fractions were pooled and normally a pool volume of around 36 ml was obtained.

The pool from the previous step was applied to a Superdex 200, prep grade (GE Healthcare, Bio-Sciences AB, Uppsala, Sweden) column (2.6 x 60 cm) equilibrated and eluted at 1 ml/min with 20 mM imidazole, 10 mM CaCl₂, 0.02 % Tween 80, 1 M glycerol, 150 mM NaCl, pH = 7.3. Fractions of 3 ml were collected and assayed for Factor VIII:C (see example 3). Factor VIII containing fractions were pooled and normally a pool volume of around 57 ml was obtained. The pool containing Factor VIII was store at - 80°C.

With the use of the above four-step purification procedure an overall yield of approximately 15 % was obtained as judged by FVIII:C and ELISA measurements.

### Example 2 Procedure for PEGylation of recombinant O-glycosylated Factor VIII

The recombinant Factor VIII molecules obtained in Example 1 are conjugated with polyethylenglycol (PEG) using the following procedure:
For the glycoPEGylation reaction to be efficient a FVIII concentration > 5mg/ml is required. Since FVIII is not normally soluble at the concentration a screening of selected buffer compositions was conducted (see table 1). Based on these considerations a buffer containing 50 mM MES, 50 mM CaCl₂, 150 mM NaCl, 20% glycerol, pH 6.0 was found to be a suitable reaction buffer.

**Table 1 Evaluation of impact of reaction conditions on FVIII solubility and aggregation.**

| **Reaction buffer composition** | **Precipitate** | **% Aggregate** |
|---|---|---|
| 10 mM Histidine, 260 mM Glycine, 1% Sucrose, 10 mM CaCl₂ | **YES** | **n.d.** |
| 50 mM HEPES, 10 mM CaCl₂, 150 mM NaCl, pH 7; | **YES** | **n.d.** |
| 50 mM MES, 10 mM CaCl2, 150 mM NaCl, pH 6.0 | **YES** | **n.d.** |
| 50 mM MES, 50 mM CaCl2, 150 mM NaCl, pH 6.0 | **NO** | **8** |
| 50 mM MES, 50 mM CaCl2, 150 mM NaCl, 10% glycerol, pH 6.0 | **NO** | **5** |
| 50 mM MES, 50 mM CaCl2, 150 mM NaCl, 20% glycerol, pH 6.0 | **NO** | **1.0-1.7** |

Recombinant FVIII which had been purified as described above was concentrated in reaction buffer either by ion exchange on a Poros 50 HQ column using step elution, on a Sartorius Vivaspin (PES) filter, 10 kDa cut-off or on an Amicon 10 kDa MWCO PES filter to a concentration of 6-10 mg/mL. The glycoPEGylation of FVIII was initiated by mixing Factor VIII (BDD) (-4.7 mg/mL final) with Sialidase (*A. ureafaciens*) (159 mU/mL), CMP-SA-glycerol-PEG-40 kDa (see WO2007/056191) (5 mol.eq.) and MBP-ST3Gal1 (540 mU) (WO 2006102652) in reaction buffer (50 mM MES, 50 mM CaCl2, 150 mM NaCl, 20% glycerol, 0.5 mM antipain, pH 6.0). The reaction mixture was incubated at 32°C until a conversion yield of ∼20-30% of total.

Following the incubation the sample was diluted with Buffer A (25 mM Tris, 5 mM CaCl₂, 20 mM NaCl, 20% glycerol, pH 7.5) and applied onto a Source 15Q column (1 cm id x 6 cm, 4.7 mL, 1 mL/min, 280 nm). The bound material was washed with Buffer A and eluted using a step gradient with Buffer B (25 mM Tris, 5 mM CaCl₂, 1 M NaCl, 20% glycerol, pH 7.5). GlycoPEGylated Factor VIII-(O)-SA-glycerol-PEG-40 kDa was eluted from the column at ∼25% Buffer B.

In order to block free galactose moieties which had been exposed on the N-glycans during the sialidase treatment the poole fraction of Factor VIII-SA-glycerol-PEG-40 kDa (1.0 mg/mL final) was mixed with CMP-SA (2,000 mol eq) and MBP-SBD-ST3Gal3 (WO 2006102652) (400 mU/mL) in reaction buffer 50 mM MES, 20 mM CaCl2, 150 mM NaCl, 10 mM MnCl2, 20% glycerol, pH 6.0 and incubated at 32°C for 11 hours.

The resulting capped, glycoPEGylated Factor VIII-SA-glycerol-PEG-40 kDa was seperated from cmp-SA and ST3Gallll by gel-filtration on a Superdex 200 column (10 cm id x 300 mm; 280 nm) equilibrated with 50 mM MES, 50mM CaCl2, 150 mM NaCl, 10 % glycerol, pH 6.0; flow rate of 0.25 mL/min. The product Factor VIII-SA-glycerol-PEG-40 kDa elutes at 38 min. The peak fraction was collected, aliquoted and subjected to subsequnt analysis.

### O-Glycan 40 kDa-GlycoPEG- BDD-FVIII Y1680F

BDD-FVIII Y1680F (1.18 mg, 0.85 mg/ml) in a buffer consisting of: imidazol (20 mM), calcium chloride (10 mM), Tween 80 (0.02 %), sodium chloride (500 mM), and glycerol (1 M) in water (pH 7.3) was thawed.

Sialidase (2.4 U, in 20 microliter buffer) from Arthrobacter ureafaciens, sialyl tranferase (His-ST3Gal-I, 2.5 mg/ml, 6.75 U, 125 microliter, EC 2.4.99.4, WO 2006102652), and cytidine monophospate *N*-5'-PEG-glycerol-neuraminic acid, CMP-SA-glycerol-PEG-40 kDa (1.9 mM, 41 microliter buffer, 78 nmol; see WO2007/056191) were added. The final volume was 1.5 ml. The resulting mixture was left for 24 hours at 32 degrees Celsius. The mixture was diluted to 20 ml with Buffer A: (Imidazol (20 mM), calcium chloride (10 mM), Tween 80 (0.02 %), and glycerol (1 M) in water (pH 7.3)).

The resulting mixture was loaded onto a MonoQ 5/50 GL column (GE Healthcare Bio-Sciences, Hillerød, Denmark). The immobilised material was washed with Buffer A (10 column volumes) after which it was eluded from the column using a gradient of: 0-100 % Buffer B (Imidazol (20 mM), calcium chloride (10 mM), Tween 80 (0.02 %), sodium chloride (1 M), and glycerol (1 M) in water (pH 7.3)) (10 CV 100 % A, 10 CV 0 - 20 % Buffer B, 10 CV 20 % Buffer B, 25 CV 20 - 100 % Buffer B, and 5 CV 100% Buffer B).

The collected material was mixed with cytidine monophospate *N*-5'acetyl-neuraminic acid (53 microgram) and sialyltransferase (MBP-SBD-ST3Gal-III, EC 2.4.99.6, see WO 2006102652). The final volume and concentrations were: 2.56 ml and 0.46 mg/ml (FVIII), 0.132 mg/ml (MBP-SBD-ST3Gal-III), and 54 micromolar (cytidine monophospate N-5'acetylneuraminic acid), respectively.

The mixture was left for 1 hour at 32 degrees Celsius at which time the mixture was diluted to 20 ml with buffer A. The resulting mixture was loaded onto a MonoQ 5/50 GL column (GE Healthcare Bio-Sciences). The immobilised material was washed with Buffer A after which it was eluded from the column using a gradient of 0-100 % (10 CV 100 % A , 10 CV 0 - 20 % Buffer B , 10 CV 20 % Buffer B, 25 CV 20 - 100 % Buffer B, and 5 CV 100% Buffer B). The protein content in the isolated fractions was evaluated using SDS-PAGE gels (Invitrogen, 7 % Tris-Acetate, NuPAGE Tris-Acetate running buffer, 70 minutes, 150 V, non-reduced conditions).

The selected fractions were pooled and concentrated using an Amicon Ultra Centrifuge Tube (Millipore, cut-off: 50 kDa). The volume after concentration was 0.5 ml. The resulting solution was loaded onto a Superose 6 10/300 GL column (GE Healthcare Bio-Sciences, Hillerød, Denmark; column volume 24 ml) that had been pre-equilibrated in a buffer consisting of: Histidine (1.5 g/l), calcium chloride (250 mg/l), Tween 80 (0.1 g/l), sodium chloride (18 g/l), and sucrose (3 g/l) in water (pH 7.0). Using the mentioned buffer and a flow of 0.6 ml/min, the components of the mixture were separated into fractions with a size of 1 ml over 1.5 column volume. The selected fractions pooled (0.015 mg/ml, 2 ml).

### Example 3 Pegylation of Y1680C with Peg-30K-Maleimide (ref. US2006/0115876 A1)

### Reagents:

1) BDD-FVIII N8-Y1680C (MW 178,000), 1200 µl, conc. 80 µg/ml, 96 µg, 0.54 nmol in buffer 20 mM imidazole, + 10 mM CaCl₂, +0.02 % Tween 80, + 1 M NaCl, 1 M gGlycerol, pH 7.3
2) Triscarboxyethylphosphine (TCEP, MW 287): 700 eq; 0.0315 µmMol; 9 µg. 1 mg TCEP was dissolved in 1 ml of buffer 20 mM limidazol, 10 mM CaCl₂, 0.02% Tween 80, 1 M Gglycerol, pH 7.3, 1 M NaCl. 109 ul of this solution was used.
3) 30 kDa PEG-maleimid (Sunbright Me-300Ma from NOF Corp., MW 29300), 10 eq., 180 µg. 4.8 mg 30 kDa PEG-maleimid was dissolved in 2.4 ml of buffer 20 mM limidazol, 10 mM CaCl₂, 0.02% Tween 80, 1 M Gglycerol, pH 7.3, 1 M NaCl. 90 uµl of this solution was used.

Buffers used for VivaP pure spin column (strong anion exchange) and Pro-spin (spin columns):
Buffer A: 20 mM Imidazol, 10 mM CaCl₂CaCl2, 0.02% Tween 80, 1M Glycerol, pH 7.3
Buffer B: 20 mM Imidazol, 10 mM CaCl₂CaCl2, 0.02% Tween 80, 1M Glycerol, pH 7.3 25mM NaCl
Buffer C: 20 mM Imidazol, 10 mM CaCl₂CaCl2, 0.02% Tween 80, 1M Glycerol, pH 7.3 50mM NaCl
Buffer D: 20 mM Imidazol, 10 mM CaCl₂CaCl2, 0.02% Tween 80, 1M Glycerol, pH 7.3 200mM NaCl
Buffer E: 20 mM Imidazol, 10 mM CaCl₂CaCl2, 0.02% Tween 80, 1M Glycerol, pH 7.3 1M NaCl

BDD-FVIIIN8 -Y1680C was thawed at room temperature and was pooled in a 5 ml tube. An amount of 109 µl of the TCEP-solution was added. The mixture was incubated at 5°C for 30min.

Next, the TCEP was removed: The reaction mixture was diluted with 42 ml of 20 mM limidazol, 10 mM CaCl₂CaCl2, 0.02% Tween 80, 1 M Gglycerol, pH 7.3, bringing the salt concentration to 31 mM. tThe solution was applied to a Viva pPure Q maxi M (Sartorius, strong anion exchange)
Equilibration: Loading 10 ml Buffer A. spin 2 min. at 2000 g (1500 RCF)
Sample loading : 4 X 11 ml of the diluted sample was loaded. For each, spin 2 min. at 2000g.
Elution buffer B (25 mM NaCl): 10 ml Buffer B was added. spin 2 min. at 2000g.
Elution buffer C (50 mM NaCl): 10 ml Buffer C was added. spin 2 min at 2000g.
Elution buffer D (200 mM NaCl): 10 ml Buffer D was added. spin 2min. at 2000g.
Elution buffer E (1 M NaCl): 2 x 750ul Buffer E was added. spin 1 min at 2000g. (repeated 2X)

The de-blocked protein was collected in the first fraction from elution with buffer E; 103 µg, 137 µg/ml as measured by Nanodrop. It was then buffer exchanged using a NAP-10 column (GE Healthcare) and 20 mM limidazol, 10 mM CaCl₂CaCl2, 0.02% Tween 80, 1 M Gglycerol, pH 7.3, 1 M NaCl as eluent. A single fraction of 1200 µl containng all the material was collected.

To this fraction was added 90 µl of the 30 kDa PEG-maleimide solution. The reaction mixture was incubated at 5°C. for a period of 20 h, after which SDS-PAGE gel analysis showed decreased intensity of the FVIII LC band and at the same time presence of a new band with increased molecular weight.

The reaction mixture was then diluted with 42 ml of buffer 20 mM limidazol, 10 mM CaCl₂CaCl2, 0.02% Tween 80, 1 M Gglycerol, pH 7.3, loaded to an Viva pure Q maxi M (strong anion exchange) spin coumn and eluted with buffers B through E as described above. An amount of 67µg protein derivatiove was collected, conc 89 µg/ml, 750 µl, which was applied a Superdex 200 10/300 column and eluted with histidine (1.5 mg/ml), CaCl₂ CaCl₂ (0.25 mg/ml), Tween 80 (0.1 mg/ml), NaCl (18 mg/ml), sucrose (3 mg/ml), pH 7. The material eluting at 9.5 to 12 min retention time was collected furnishing the mono PEGylated BDD-FVIII N8-Y1680C mutant, 35 µg, 14 µg/ml. SDS-PAGE analysis of the fractions showed correct structure of the compound, with the PEG attached to the LC.

Using the above protocol, a monoPEGylated BDD-FVIII Y1680C compound was prepared. In this compound the PEG-group is attached to the 1680 position in BDD-FVIII. This particular modification gives rise to two simultaneous effects: a reduced vWF binding and PEG-mediated half-life extension.

### Example 4: FVIII:C measured in chromogenic assay

The FVIII activity (FVIII:C) of the rFVIII compound was evaluated in a chromogenic FVIII assay using Coatest SP reagents (Chromogenix) as follows: rFVIII samples and a FVIII standard (e.g. purified wild-type rFVIII calibrated against the 7th international FVIII standard from NIBSC) were diluted in Coatest assay buffer (50 mM Tris, 150 mM NaCl, 1 % BSA, pH 7.3, with preservative). Fifty µl of samples, standards, and buffer negative control were added to 96-well microtiter plates (Nunc) in duplicates. The factor IXa/factor X reagent, the phospholipid reagent and CaCl₂ from the Coatest SP kit were mixed 5:1:3 (vol:vol:vol) and 75 µl of this added to the wells. After 15 min incubation at room temperature 50 µl of the factor Xa substrate S-2765/thrombin inhibitor I-2581 mix was added and the reactions incubated 10 min at room temperature before 25 µl 1 M citric acid, pH 3, was added. The absorbance at 415 nm was measured on a Spectramax microtiter plate reader (Molecular Devices) with absorbance at 620 nm used as reference wavelength. The value for the negative control was subtracted from all samples and a calibration curve prepared by linear regression of the absorbance values plotted vs. FVIII concentration. The specific activity was calculated by dividing the activity of the samples with the protein concentration determined by HPLC. The concentration of the sample was determined by integrating the area under the peak in the chromatogram corresponding to the light chain and compare with the area of the same peak in a parallel analysis of a wild-type unmodified rFVIII, where the concentration was determined by amino acid analyses. The data in table 1 demonstrate that the specific FVIII:C activity was maintained for the O-glycoPEGylated rFVIII compounds.

### Example 5: FVIII:C measured in one-stage clot assay

FVIII:C of the rFVIII compounds was further evaluated in a one-stage FVIII clot assay as follows: rFVIII samples and a FVIII standard (e.g. purified wild-type rFVIII calibrated against the 7th international FVIII standard from NIBSC) were diluted in HBS/BSA buffer (20 mM hepes, 150 mM NaCl, pH 7.4 with 1 % BSA) to approximately 10 U/ml followed by 10-fold dilution in FVIII-deficient plasma containing VWF (Dade Behring). The samples were subsequently diluted in HBS/BSA buffer. The APTT clot time was measured on an ACL300R or an ACL5000 instrument (Instrumentation Laboratory) using the single factor program. FVIII-deficient plasma with VWF (Dade Behring) was used as assay plasma and SynthASil, (HemosIL™, Instrumentation Laboratory) as aPTT reagent. In the clot instrument, the diluted sample or standard is mixed with FVIII-deficient plasma, aPTT reagents at 37°C. Calcium chloride is assed and time until clot formation is determined by turbidity. The FVIII:C in the sample is calculated based on a standard curve of the clot formation times of the dilutions of the FVIII standard. The data in table 1 demonstrate the ratio between clotting and chromogenic activity.

**Table 1 Specific chromogenic activity and clotting activity relative to the chromogenic activity.**

| **GlycoPEGylated N8 compound** | **Specific chromogenic activity (IU/mg)** | **Ratio between clotting and chromogenic activity** |
|---|---|---|
| **N8** | 11 819 ± 727 (5) | 1.02 ± 0.12 (3) |
| **10K-PEG-[O]-N8** | approx 8331 (1) | 1.01 ± 0.10 (3) |
| **40K-PEG-[O]-N8** | 9760 ± 886 (8) | 0.78 ± 0.06 (3) |
| **80K-PEG-[O]-N8** | 12129 ± 2643 (3) | 0.45 ± 0.05 (3) |
| **80K-PEG-[N]-N8** | 8819 ± 1685 (3) | 0.46 (1) |
| **(80K-PEG)₂-[N]-N8** | 3058 ± 318 (2) | 0.32 (1) |
| **(40K-PEG)₂-[N,O]-N8** | 10077 ± 1089 (2) | 0.31 ± 0.05 (3) |
| **40K-PEG-[O]-FVIII (Y1680F)** | 4901 (1) | 1.59 (1) |
| **30K-PEG-FVIII (Y1680C)** | 8736 ± 600 (2) | 0.80 ± 0.05 (2) |

### Example 6: Pharmacokinetics of rFVIII in FVIII- and VWF-deficient mice

The phamacokinetics of rfviii variants were evaluated in FVIII-deficient mice (FVIII exon 16 knock out (KO) mice with c57bl/6 background, bred at Taconic m&b) or in vWF-deficient mice (vWF exon 4 + 5 KO mice with c57bl/6 background bred at Charles River, Germany). The vWF-KO mice had 13% of normal FVIII:C, while the FVIII-KO mice had no detectable FVIII:C. A mixture of male and female (approximately1:1) with an approximate weight of 25 grams and age range of 16-28 weeks were used. The mice received a single i.v. Injections of rFVIII (280 iu/kg) in the tail vein. Blood was taken from the orbital plexus at time points up to 64 hours after dosing using non-coated capillary glass tubes. Three samples were taken from each mouse, and 2 to 4 samples were collected at each time point. Blood was immediately stabilized with sodium citrate and diluted in four volumes FVIII coatest sp buffer (see example 4) before 5 min centrifugation at 4000 × g. Plasma obtained from diluted blood was frozen on dry ice and kept at -80°c. The FVIII:C was determined in a chromogenic assay as described in example 4. Pharmacokinetic analysis was carried out by non-compartmental methods (NCA) using winnonlin pro version 4.1 software. Table 2 show estimates for the pharmacokinetic parameters: the half-life (t½), clearance (cl) and mean residence time (MRT). The data show than the clearance was decreased and the half-life and the mean residence time increased upon PEGylation.

BDD-FVIII was cleared fast in vWF KO mice (T_{½} of 0.5 h), which corresponds with the generally accepted hypothesis of vWF being a carrier and stabiliser for FVIII in the circulation. When vWF KO mice was i.v. administered with 10K-80K glycoPEGylated BDD-FVIII an increase in the terminal T_{½} was observed (in the range of 2.7 to 11 h), corresponding to a 5-22 fold increase, as compared to BDD-FVIII (Table 2), suggesting that glycoPEGylation decreased the importance of FVIII binding to vWF. This is in agreement with preliminary data obtained of the BDD-FVIII-PEG-vWF binding, which showed a reduced binding to vWF of BDD-FVIII as the attached PEG-moiety increased.

**Table 2 Pharmacokinetic parameters estimates of FVIII glycoPEGylated with different sizes of PEG after i.v. administration to vWF KO mice based on ELISA**

| ***Compound*** | ***FVIII dose (IU*/*kg)*** | ***T_{½} (h)*** | ***CL (ml*/*h*/*kg)*** | ***MRT (h)*** |
|---|---|---|---|---|
| **N8** | 280 | 0.45-0.53 | 151-186 | 0.6 |
| **10K-PEG-[O]-N8** | 280 | 2.7 | 52.1 | 2.5 |
| **40K-PEG-[O]-N8** | 280 | 8.3 | 13.8 | 9.3 |
| **(40K-PEG)₂-[N,O]-N8** | 280 | 11 | 5.7 | 17.3 |
| **80K-PEG-[N]-N8** | 280 | 9.8 | 8.7 | 11.6 |

Interestingly, glycoPEGylation almost "normalised" the exposure profile of BDD-FVIII in vWF KO mice as compared to FVIII KO mice. The larger the PEG group attached, the smaller the difference between the pharmacokinetics in FVIII KO and vWF KO mice. The ratio between the half-lifes of the glycoPEGylated BDD-FVIII variants were decreasing with the size of the PEG group increased. This indicates that exposure of the glycoPEGylated variants is less dependent of vWF as compared to the unmodified BDD-FVIII. A similar trend can be observed i FVIII KO mice when using FVIII variants lacking vWF binding (Table 3).

**Table 3 Pharmacokinetic parameters estimates of FVIII glycoPEGylated with different sizes of PEG after i.v. administration to FVIII KO mice based on activity**

| ***Compound*** | ***FVIII dose (IU*/*kg)*** | ***T_{½} (h)*** | ***CLml*/*h*/*kg)*** | ***MRT (h)*** |
|---|---|---|---|---|
| **40K-PEG-O-F8-500-Y1680F** | 280 | 6.13 | 12.30 | 8.06 |
| **N8** | 280 | 6.1-7.8 | 6.5-11 | 9.4-11 |
| **F8-500-Y1680F** | 280 | 0.48 | 217 | 0.65 |
| **F8-500-Y1680C 30K-PEG** | 240 | 4.36 | 34.9 | 4.73 |
| **40K-PEG-O-N8** | 280 | 12-16 | 3.0-4.4 | 16-22 |

### Example 7 Haemostatic effect of FVIII variants lacking the ability to bind vWF in a FeCl₃ induced injury model in haemophilia A mice

Mice were anesthetized with a mixture of Ketamine (Ketaminol® Vet. (Intervet); 100 mg/kg), Xylazine (Narcoxyl® Vet. Injection solution (Intervet); 5.6 mg/kg), and Atropine (Atropine Sulfate (Phoenix Pharma); 0.3 mg/kg) and placed on a heating pad (37°C) to maintain body temperature. The carotid artery was subsequently exposed and a 0.5PSB Nanoprobe was placed around the artery. Test compounds were injected into the laterals tail vein 5 minutes before induction of the FeCl₃ injury. FeCl₃ injury was induced by placing a filter paper (2 x 5 mm) briefly soaked in a 10 % FeCl₃ solution around the artery adjacent to the probe and removed after 3 min. The artery was washed three times with 0.9% NaCl and finally Surgilube (an acoustic coupler) was applied in order to displace air in the flow probe and secure an optimised measurement of the blood flow after removal of the FeCl₃ saturated filter paper. Mice were excluded if the initial blood flow was too low (below 0.4 ml/min) or if flow could not be measured after removal of the FeCl₃. Blood flow (ml/min) was recorded for 25 min after removing the FeCl₃ saturated filter paper using the software Chart5 (version 5.5.5.20) from AD instruments.

### Data analysis

Data are presented as mean ± SEM unless otherwise stated and were analysed using GraphPad Prism version 5 (La Jolla, CA, USA). The effect of the two compounds was compared using one-way ANOVA followed by Tukey's test for multiple comparisons. P < 0.05 was considered statistically significant.

### Results

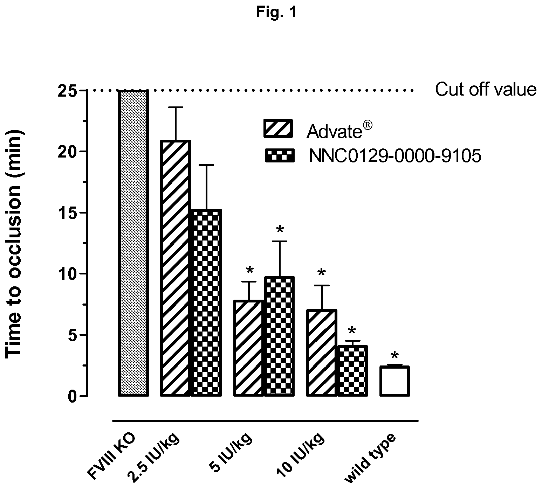

After FeCl₃ injury, all the vessels in mice with a normal functioning coagulation system (C57BL/6NTac) occluded within 3.3 minutes with a mean time to occlusion of 2.4 ± 0.2 minutes (n=6; Figure 1). None of the FVIII knockout mice occluded within the 25 minutes observation period (n=6). NNC 0129-0000-9105 dose dependently reduced the time to occlusion (NNC 0129-0000-9105 corresponds to: F8-500 Y1680F 40 kDa-PEGyleret on the O-linked glycan in the truncated B domain). The time to occlusion was significantly shorter after treatment with either 5 or 10 IU/kg compared to vehicle treated animals. A similar effect of Advate® was observed and there was no statistic difference between the effect of Advate® and NNC 0129-0000-9105 (Figure 1).

### Example 8 Haemostatic effect of FVIII variants lacking the ability to bind vWF in a tail transaction bleeding model in haemophilia A mice

Mice were anesthetized with pentobarbital (100 mg/kg, i.p.; Nomeco, Roskilde, Denmark) and placed on a heating pad to maintain body temperature. Bleeding was induced by amputating 4 mm of the tip of the tail with a sharp scalpel. Ten minutes prior to amputation, the tail was placed in a test tube containing 14 mL of saline (37° C) and 5 min before amputation, the test compounds were administered (20 or 280 U/kg) by tail vein injection in a volume of 5 mL/kg. Haemophilia A mice and animals with a normal coagulation were treated with the same volume of vehicle. Blood was collected over a 30 minutes period where after the animals were euthanized. Blood loss was determined by quantifying the amount of hemoglobin. Thus, erythrocytes were isolated by centrifugation at 4000x for 5 min. The supernatant was discarded and the cells lysed with hemoglobin reagent (ABX Lysebio; HORIBA, ABX, Roedover, Denmark). Cell debris was removed by centrifugation at 4000 x g for 5 min. Samples were read at 550 nm and the total amount of hemoglobin was determined from a standard curve (Hemoglobin standards; J.T. Baker 3074; Bie & Berntsen, Roedover, Denmark).

### Data analysis

Data are presented as mean ± SEM unless otherwise stated and were analysed using GraphPad Prism version 5 (La Jolla, CA, USA). The effect of the two compounds was compared using one-way ANOVA followed by Tukey's test for multiple comparisons. P < 0.05 was considered statistically significant.

### Results

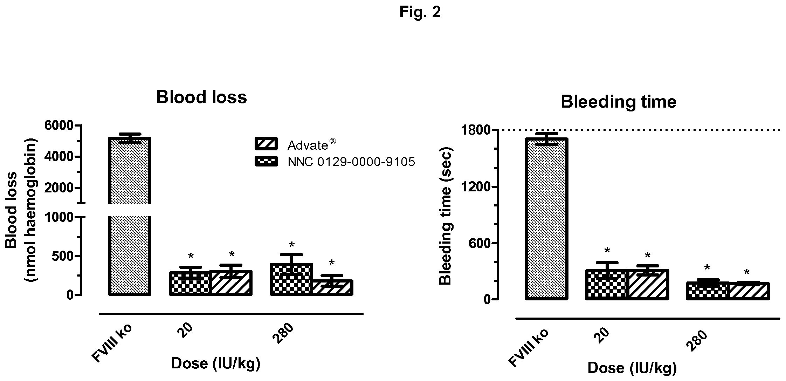

The dose response relationship for Advate® in the tail bleeding model in FVIII knockout mice has previously been investigated thoroughly (Documentum ObjectID: 0900c76e80e3e7a5). 200 IU/kg Advate® normalised both the blood loss and bleeding time and the ED₅₀ value was 39 and 28 IU/kg for the blood loss and bleeding time, respectively. To test the haemostatic effect of NNC 0129-0000-9105 the effect of 20 and 280 IU/kg was compared to the same doses of Advate®. NNC0129-0000-9105 (20 and 280 IU/kg) significantly reduced the blood loss and bleeding time compared to the vehicle treated animals (Figure 2). No differences were observed between the effect of Advate® and NNC 0129-0000-9105.

The effect of 20 IU/kg of Advate® was more pronounced than expected from the previous study (Documentum ObjectID: 0900c76e80e3e7a5). This is most likely due to a combined effect caused by differences in the actually dosed activity of Advate® and the general variation in the tail bleeding mode. Thus, the intention was to dose 20 IU/kg in both studies, however, an analysis of the FVIII activity (Chromogenix®) in the dosing solution showed that the activity was 20% higher (ELN 15876-199) and 15% lower than indented in the current and previous study, respectively. This does however, not changes the overall conclusion of the study that NNC 0129-0000-9105 and Advate® have comparable effect since both compounds were dosed with comparable FVIII activity in the current study.

### Example 9 Generation of FVIII variants with platelet binding properties which does not bind to vWF.

The variable regions of the heavy and light chain of the anti-GPIIa/IIIB antibody, AP3 were amplified from RNA isolated from hybridoma cells expressing the AP3 antibody, using the SMART™ RACE cDNA Amplification Kit (Clontech, Ca). The primers used for the amplification of the variable regions of the two AP3 chains were:
**Heavy chain:**
   Universal Primer Mix A (Clontech, CA):
      Long (0.4 µM):
         5'-ctaatacgactcactatagggcAAGCAGTGGTATCACGCAGAGT-3' (SEQ ID NO 5)
      Short (2 µM):
         5'-ctaatacgactcactatagggc-3' (SEQ ID NO 6)
      Primer 69 (10 µM)
         5'-gctctagactaacactcattcctgttgaagctcttg-3' (SEQ ID NO 7)
**Light chain**
   Universal Primer Mix A (Clontech:
      Long (0.4 µM):
         5'-ctaatacgactcactatagggcAAGCAGTGGTATCACGCAGAGT-3' (SEQ ID NO 8)
      Short (2 µM):
         5'-ctaatacgactcactatagggc-3' (SEQ ID NO 9)
      Primer 312 (10 µM)
         5'-gtctaccacaacacacgtgac-3' (SEQ ID NO 10)

The variable regions were cloned into the pCR4 vector using the Zero Blunt® TOPO® PCR Cloning Kit for Sequencing (- Cat. No. K287520 from Invitrogen, CA). The variable regions were subsequently subcloned into a murine IgG1 frameworks in pTT5 based expression vectors. The two chains encoding the full length AP3 ab was transiently expressed in Hek293 6E cells. Binding of the full length antibody to resting platelets was confirmed by FACS analysis.

Based on the DNA sequences encoding the variable regions of the light and heavy chains of the AP3 antibody two single chain antibody formats of AP3 ab (AP3 LC-HC scFV and AP3 HC-LC scFV) were generated (SEQ 1 and SEQ 2). The two single chain formats of the AP3 ab were subsequently subcloned into pTT5 based expression vectors. The binding of the two single-chain antibodies to GPIIa/IIIB of resting platelets was confirmed by FACS analysis.

Fusionproteins between AP3 LC-HC scFV or AP3 HC-LC scFV and B-domain deleted - a3 domain deleted FVIII were made by inserting AP3 LC-HC scFV or AP3 HC-LC scFV between aa R761 and Q1686 of BDD FVIII (SEQ 3 and SEQ 4 respectively) using standard molecular biology methods.

### SEQ ID NO 11: AP3-LC-HC scFV-FLAG

DIVMTQAAPSVPVTPGESVSISCRSSRSLLHSNGNTYLCWFLQRPGQSPQLLI-
YRMSNLASGVPDRFSGSGSGTAFTLRISRVEAEDVGVYYCMQH-
LEYPFTFGSGTKLEIKRGGGGSGGGGSGGGGSQVQLQQSGAELVRPGTSVKISCK-
ASGYTFTNYWLGWVKQRPGHGLEWIGDIYPGGGYNKYNENFKGKATLTADTSSSTAY-
MQLSSLTSEDSAVYFCAREYGNYDYAMDSWGQGTSVTVSSDYKDDDDK*

### SEQ ID NO 12: AP3-HC-LC scFV-FLAG

QVQLQQSGAELVRPGTSVKISCKASGYTFTNYWLGWVKQRPGHGLEWIGDIYPGGGYN-
KYNENFKGKATLTADTSSSTAYMQLSSLTSEDSAVYFCAREYG-
NYDYAMDSWGQGTSVTVSSGGGGSGGGGSGGGGSDIVMTQAAPSVPVTPGESVSIS-
CRSSRSLLHSNGNTYLCWFLQRPGQSPQLLIYRMSNLASGVPDRFSGSGSGTAFT-
LRISRVEAEDVGVYYCMQHLEYPFTFGSGTKLEIKRDYKDDDDK*

Based on these constructs the variant AP3 scFv LC-HC C34S S248C could be generated using standard molecular biology methods.

### Generation of N-((3-(ω-(3-(2,5-dioxo-2,5-dihydropyrrol-1-yl)propionylamino)10 kDa PEGyl)propionylamino)acetyl)-O²-[5']cytidylyl-ξ-neuraminic acid

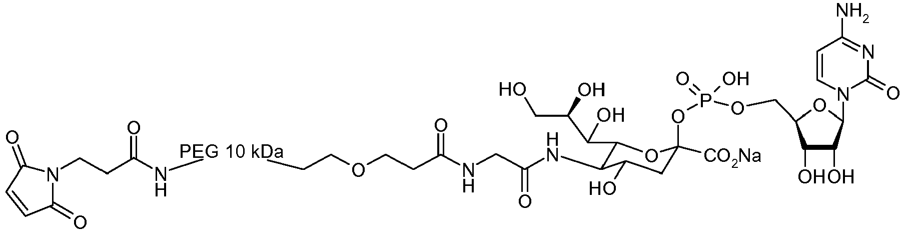

### Step 1:

### 3-(2,5-Dioxo-2,5-dihydropyrrol-1-yl)propionic acid 2,5-dioxopyrrolidiny-1-yl ester

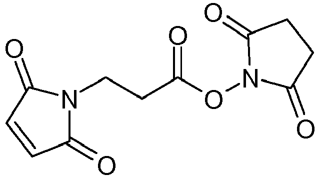

3-Maleimidopropionic acid (1.0 g, 5.9 mmol) was dissolved in tetrahydrofuran (20 ml). 2-Succinimido-1,1,3,3-tetramethyluronium tetrafluoroborate (TSTU, 2.14 g, 7.1 mmol) and ethyldiisopropylamine (1.24 ml, 7.1 mmol) were added subsequently. *N,N-*Dimethylformamide (5 ml) was added. The reaction mixture was stirred at room temperature, while it was turning sluggish. The mixture was stirred for 2 min. *N*,*N*-Dimethylformamide (5 ml) was added. The mixture was stirred for 2.5 h at room temperature. It was diluted with dichloromethane (150 ml) and was washed subsequently with a 10% aqueous solution of sodium hydrogensulphate (150 ml), a saturated aqueous solution of sodium hydrogencarbonate (150 ml) and water (150 ml). It was dried over magnesium sulphate. The solvent was removed in vacuo. The crude product was recrystallized from ethyl acetate to give 1.20 g of 3-(2,5-dioxo-2,5-dihydropyrrol-1-yl)propionic acid 2,5-dioxopyrrolidiny-1-yl ester.
MS: m/z = 289, required for [M+Na]⁺: 289
¹H-NMR (CDCl₃) δ 2.82 (m, 4 H); 3.02 (t, 2 H); 3.94 (t, 2 H), 6.73 (s, 2 H).

### Step 2:

*N*-((3-(ω-Amino10 kDa PEGyl)propionylamino)acetyl)-*O*²-[5']cytidylyl-ξ-neuraminic acid (100 mg, 0.009 mmol) was dissolved in a mixture of tetrahydrofuran (2 ml) and dichloromethane (10 ml). A solution of 3-(2,5-dioxo-2,5-dihydropyrrol-1-yl)propionic acid 2,5-dioxopyrrolidiny-1-yl ester (50 mg, 0.18 mmol) in dichloromethane (3 ml) was added. Ethyldiisopropylamine (0.005 ml, 0.028 mmol) was added. The reaction mixture was stirred at room temperature fro 16 h. Dichloromethane (2 ml) and ethyldiisopropylamine (0.5 ml) were added. Amionomethylated polystyrene resin (commercially available at e.g. Novabiochem, loading 0.85 mmol/g, 438 mg, 0.372 mmol) was added. The mixture was slowly stirred at room temperature for 1 h. The resin was removed by filtration. The solvent was removed in vacuo with a bath temperature of 25 °C. The residue was dissolved in dichloromethane (4 ml). Ether (200 ml) was added. The mixture was left at room temperature for 2 h in order to let the formed precipitation grow old. The precipitation was isolated by filtration and dried in vacuo to give 38 mg of the title compound. The ¹H-NMR spectrum in DMSO-d₆ showed the presence of a maleimide group.

### Conjugation of AP3 scFv LC-HC C34S S248C to FVIII (NNC 0129-0000-3270)

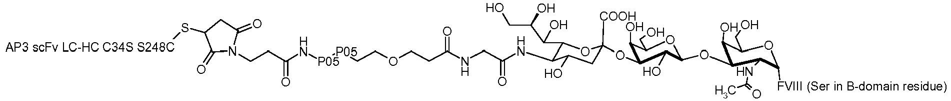

### Step 1:

### Attachment of N-((3-(ω-(3-(2,5-dioxo-2,5-dihydropyrrol-1-yl)propionylamino)10 kDa PEGyl)-propionylamino)acetyl)-O²-[5']cytidylyl-ξ-neuraminic acid to Cys 248 of AP3 scFV C24S S248C

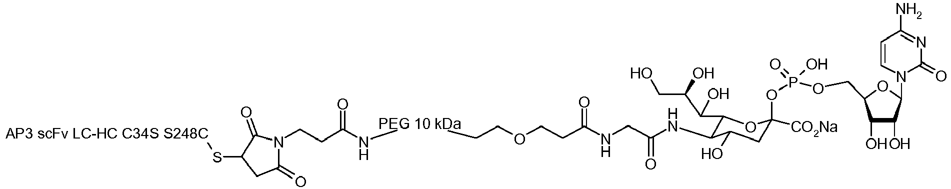

A solution of Tris(2-carboxyethyl)phosphine hydrochloride (0.40 mg) in a buffer (0.40 ml)consisting of 20 mM imidazole, 10 mM CaCl₂, 0.02% Tween80, 1 M glycerol which had been adjusted to pH 7.35 was added to a solution with a concentration of 0.53 mg/ml of AP3 scFv LC-HC C34S S248C with a Flag tag at its C-terminus and an extrac Cys attached to the Cysteine at position 248 via a S-S bridge in a solution of 100 mM HEPES and 150 mM NaCl which had been adjusted to pH 7.5 with (4 ml, 2.12 mg, 76 nmol). The reaction mixture was gently shaken at 20 °C. It was divided into two parts. Each of them were added to a PD-10 column (GE-Healthcare), using a buffer of 25 mM HEPES which had been adjusted to pH 7.0. The eluates of the column (each of them 3.5 ml) were combined.

A solution of *N*-((3-(ω-(3-(2,5-dioxo-2,5-dihydropyrrol-1-yl)propionylamino)10 kDa PEGyl)propionylamino)acetyl)-*O*²-[5']cytidylyl-ξ-neuraminic acid (3.3 mg, 305 nmol) in a buffer of 25 mM HEPES, which had been adjusted to pH 7.0 (0.43 ml) was added to the solution of the protein. The reaction mixture was gently shaken at 20 °C for 4 h. It was placed in an Amicon ultracentrifugation device with a cut off of 10 kDa. It was subjected to an ultracentrifugation at 4000 rpm at 10 °C for 10 min. The mixture was kept in the freezer until purification.

For purification the mixture was thawed. It was subjected to a size exclusion chromatography utilizing a Superdex 200 gel with a bed size of 16 mm in diameter and 60 cm in length at a flow of 1 ml/min and a buffer of 25 mM TRIS and 150 mM NaCl which had been adjusted to pH 8.0. The fractions containing the desired product were pooled to give 1.61 mg of the desired protein. The SDS-PAGE gel was in accordance with the expectation.

### Step 2:

A solution of B-domain deleted FVIII which has a residual B-domain sequence of SFSQNSRHPSQNPPVLKRHQR at the C-terminus of the heavy chain (1 mg, 5.64 mmol) in a buffer consisting of 20 mM imidazole, 10 mM CaCl₂, 150 mM NaCl, 0.02% Tween80 and 1 M glycerol, which had been adjusted to pH 7.35 (0.018 ml) was placed in an Amicon ultracentrifugation device with a cut off of 10 kDa. A solution of the attachment product of N-((3-(ω-(3-(2,5-dioxo-2,5-dihydropyrrol-1-yl)propionylamino)10 kDa PEGyl)propionylamino)-acetyl)-*O*²-[5']cytidylyl-ξ-neuraminic acid to Cys 248 of AP3 scFV C24S S248C (1.29 mg, 34 nmol) in a solution of 25 mM TRIS and 150 mM NaCl which had been adjusted to pH 8.0 (0.680 ml) was added and a buffer consisting of 20 mM histidine, 10 mM CaCl₂, 10% glycerol, 0.02% Tween80, 500 mM NaCl which had been adjusted to pH 6.07 (3 ml) were added subsequently. The mixture was subjected to an ultracentrifugation at 4000 rpm at 10 °C for 20 min. The remaining volume was 0.800 ml or 1.25 mg/ml for FVIII. A solution of Sialidase from A. Urifaciens (0.43 mg/ml, 302 U/mg, 0.0049 ml, 0.645 U) and a solution of ST3-Gal-I (2.5 mg/ml, 0.105 mg, 0.042 ml) were added subsequently. The reaction mixture was gently shaken at 32 °C for 1 min and thereafter left at 32 °C for 18 h. It was kept in the freezer until purification.

The reaction mixture was thawed. It was divided into two parts, each of which were subjected to a size exclusion chromatography using a Superose 6 gel with a bed size of 10 mm in diameter and 300 mm in length at a flow of 0.30 ml/min and a buffer consisting of 20 mM imidazole, 10 mM CaCl₂, 0.02% Tween80, 150 mM NaCl, and 1 M glycerol, which had been adjusted to pH 7.35 as eluent. All fractions of both runs containing the desired product were pooled. They were placed in an Amicon ultracentrifugation device with a cut off of 10 kDa and subjected to an ultracentrifugation at 4000 rpm at 10 °C for 18 min.

A solution of CMP-*N*-acetylneuraminic acid (CMP Neu*N*ac, 1.5 mg, 2597 nmol) in a buffer consisting of 20 mM imidazole, 10 mM CaCl₂, 0.02% Tween80, 150 mM NaCl, and 1 M glycerol, which had been adjusted to pH 7.35 (0.100 ml) and a 0.33 mg/ml solution of ST3Gal-III (0.10 ml, 0.033 mg) were added subsequently. The reaction mixture was gently shaken at 300 rpm and thereafter kept in the freezer until purification.

The reaction mixture was divided into two parts. Each of those was filtered through a 0.00045 mm filter. They were applicated to a sepharose column with a bed size of 5 mm in diameter and 5 cm in length to which a F25 antibody had been attached after activation with CNBr. F25 is a known antibody for FVIII. After application, the column was washed for 3 CV with a buffer consisting of 20 mM imidazole, 10 mM CaCl₂, 0.02% Tween80, 150 mM NaCl, and 1 M glycerol, which had been adjusted to pH 7.35 at a flow of 0.6 ml/min. Thereafter it was washed for 3 CV with a buffer consisting of 20 mM imidazole, 10 mM CaCl₂, 0.02% Tween80, and 650 mM NaCl, which had been adjusted to pH 7.35 at a flow of 0.6 ml/min. Finally, the compound was eluted within 6 CVwith a buffer consisting of 20 mM imidazole, 10 mM CaCl₂, 0.02% Tween80, 2.5 M NaCl in a 50%v/v ethylene glycol/water solution, which had been adjusted to pH 7.35 at a flow of 0.1 ml/min. The fractions of both runs containing the desired product were pooled. They were placed in an Amicon ultracentrifugation device with a cut off of 10 kDa. They were subjected to an ultracentrifugation at 4000 rpm at 10 °C for 14 min. The remaining solution was subjected to a size exclusion chromatography on a Superose 6 material with a bed size of 10 mm in diameter and 30 cm in length with a flow of 0.50 ml/min utilizing a buffer consisting of 10 mM Histidine, 1.7 mM CaCl₂, 0.01% Tween80, 0.3 M NaCl, 8.8 mM sucrose which had been adjusted to pH 7. The fractions containing the desired compound in a suitable purity were pooled and place in an Amicon ultracentrifugation device with a cut off of 10 kDa. They were subjected to an ultracentrifugation at 4000 rpm at 9 °C for 12 min. Using a molar absorbance of 14.46, the yield was found to be 0.0176 mg of a conjugate of AP3 scFv LC-HC C34S S248C to FVIII. The analysis by SDS-PAGE gel under non-reduced conditions were in accordance with the expectation.

### Generation of N-((3-(ω-(4-formylbenzoylamino)10 kDa PEGyl)propionylamino)acetyl)-O²-[5']cytidylyl-ξ-neuraminic acid

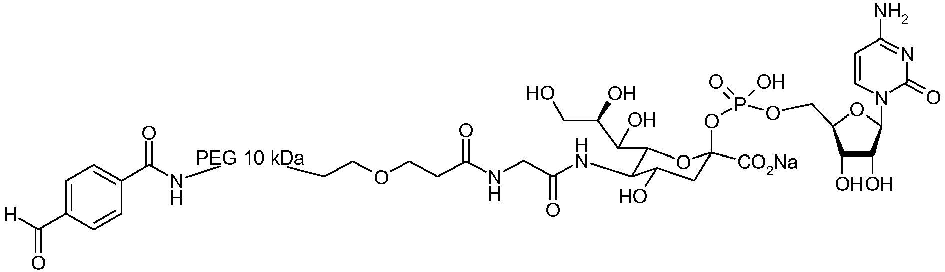

### Step 1:

### N-((3-(ω-(9H-Fluoren-9-ylmethoxycarbonylamino)10 kDa PEGyl)propionylamino)acetyl)-O²-[5']cytidylyl-ξ-neuraminic acid (NNC 0129-0000-3219)

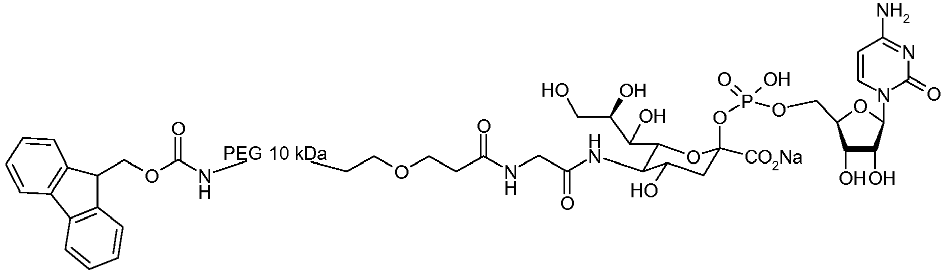

*N*-(aminoacetyl)-*O*²-[5']cytidylyl-ξ-neuraminic acid (18 mg, 0.029 mmol) was dissolved in a buffer consisting of 50 mM TRIS which had been adjusted to pH 8.9 (4 ml). The current pH was checked and was adjusted to pH 8.9 by addition of 0.1 N hydrochloric acid. THF (16 ml) was added. Approximately half of the final amount of 3-(ω-(9H-fluoren-9-ylmethoxycarbonylamino)10 kDa PEGyl)propionic acid N-hydroxysuccinimidyl ester (commercially available at for example at Rapp Polymere GmbH, 200 mg in total, 0.019 mmol) was added. The reaction mixture was stirred at room temperature. After 1 h, the second half of 3-(ω-(9H-fluoren-9-ylmethoxycarbonylamino)10 kDa PEGyl)propionic acid N-hydroxysuccinimidyl ester was added. The reaction mixture was stirred for 16 h at room temperature. The THF was removed in vacuo with a bath temperature of 25 °C. The remaining mixture was filtered and subjected to a size exclusion chromatography, using a G25 gel with a bed size of 26 mm in diameter and 10 cm in length at a flow of 7 ml/min, utilizing a buffer of 25 mM ammonium hydrogencarbonate. The fractions containing the desired compound were pooled and lyophilized to give 453 mg of material containing *N*-((3-(ω-(9H-fluoren-9ylmethoxycarbonylamino)10 kDa PEGyl)propionylamino)acetyl)-*O*²-[5']cytidylyl-ξ-neuraminic acid.

The ¹H-NMR-spectrum performed in DMSO-d₆ showed the presence of the cytidylyl moiety as well as the fluorenyl-9-ylmethoxycarbonyl moiety. The material was stored in the freezer.

This reaction was repeated with 1 g of 3-(ω-(9H-fluoren-9-ylmethoxycarbonylamino)10 kDa PEGyl)propionic acid N-hydroxysuccinimidyl ester and 90 mg of *O*²-[5']cytidylyl-ξ-neuraminic acid. The purification was performed on a HPLC C4-column with a diameter of 2 cm, using a gradient of 30-50% of a mixture consisting of a 5 mM aqueous solution of ammonium hydrogencarbonate in acetontrile in a 50 mM solution of ammonium hydrognecarbonate in water. The fractions containing the desired compound were collected and lyophilized to give 288 mg of the desired compound. The ¹H-NMR spectrum corresponded to the ¹H-NMR spectrum found in the experiment described above.

### Step 2:

### N-((3-(ω-Amino10 kDa PEGyl)propionylamino)acetyl)-O²-[5']cytidylyl-ξ-neuraminic acid (NNC 0129-0000-3227)

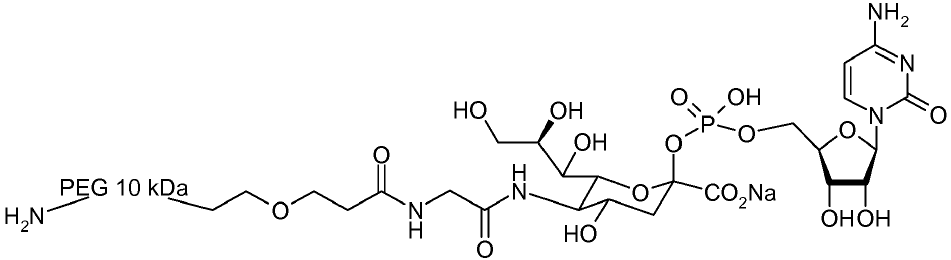

*N*-((3-(ω-(9H-Fluoren-9-ylmethoxycarbonylamino)10 kDa PEGyl)propionylamino)acetyl)-*O*²-[5']cytidylyl-ξ-neuraminic acid (453 mg) were dissolved in N,N-dimethylformamide (12 ml). Piperidine (1.25 ml) was added. The clear solution was stirred for 20 min at room temperature. Ether (200 ml) was added. The mixture was left at room temperature for 1.5 h, in order to let the formed precipitation grow old. The precipitation was isolated by riltration. It was dissolved in dichloromethane (4 ml). Ethyldiisopropylamine (1 ml) was added. Ether (250 ml) was added. The mixture was left at room temperature for 16 h in order to let the formed precipitation grow oled. The precipitation was isolated by filtration and dried in vacuo. The ¹H-NMR spectrum in DMSO-d₆ showed no signals of a 9H-fluoren-9-ylmethoxycarbonyl group, whereas signals assigned to a cytidylyl moiety could be found. The material was stored in the freezer.

### Step 3:

### 4-Formylbenzoic acid 2,5-dioxopyrrolidin-1-yl ester

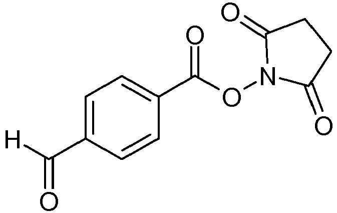

Triethylamine (2.04 ml, 14.65 mmol) and 2-succinimido-1,1,3,3-tetramethyluronium tetrafluoroborate (TSTU, 4.44 g, 14.65 mmol) were successively added to a solution of 4-formylbenzoic acid (2.0 g, 13.3 mmol) in *N*,*N*-dimethylformamide (30 ml). The reaction mixture was stirred at room temperature for 16 h. It was diluted with ethyl acetate (150 ml) and washed with a 10% aqueous solution of sodium hydrogen sulphate (100 ml). The aqueous phase was extracted with ethyl acetate (2 x 30 ml). The combined organic layers were washed with a mixture of brine (50 ml) and water (50 ml). The combined organic layers were dried over magnesium sulphate. The solvent was removed in vacuo. The crude product was recrystallized from ethyl acetate to give 1.89 g of 4-formylbenzoic acid 2,5-dioxopyrrolidin-1-yl ester.
¹H-NMR (CDCl₃). δ 2.95 (s, 4 H); 8.04 (d, 2 H), 8.32 (d, 2 H); 10.15 (s, 1 H).

### Step 4:

*N*-((3-(ω-Amino10 kDa PEGyl)propionylamino)acetyl)-*O*²-[5']cytidylyl-ξ-neuraminic acid (42 mg, 0.004 mmol) was dissolved in dichloromethane (2 ml). Ethyldiisopropylamine (0.002 ml, 0.012 mmol) was added to the solution. A solution of 4-formylbenzoic acid 2,5-dioxopyrrolidin-1-yl ester (19.32 mg, 0.078 mmol) was in dichloromethane (0.5 ml) was added. The reaction mixture was stirred for 16 h at room temperature. The solvent was removed in vacuo with a bath temperature of 25 °C. The residue was suspended in a 25 mM aqueous solution of ammonium hydrogencarbonate (15 ml). The non-soluble material was removed by filtration. It was divided into 5 parts. Each of them were subjected to a size exclusion chromatography using a G25 on a column with diameter of 26 mm and a length of 10 cm with a flow of 7 ml/min utilizing a buffer of 25 mM ammonium hydrogencarbonate. All the fractions containing the desired material were combined and lyophilized. The ¹H-NMR spectrum in DMSO-d6 showed the presence of both the aldehyde moiety and the cyidylyl moiety. The obtained material was kept in the freezer.

### Conjugation of Abciximab to FVIII at the O-glycan (NNC 0129-0000-3279)

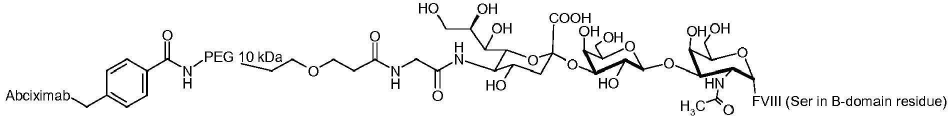

### Step 1:

### Conjugation of N-((3-(ω-(4-formylbenzoylamino)10 kDa PEGyl)propionylamino)acetyl)-O²-[5']cytidylyl-ξ-neuraminic acid to one N-terminus of Abxicimab

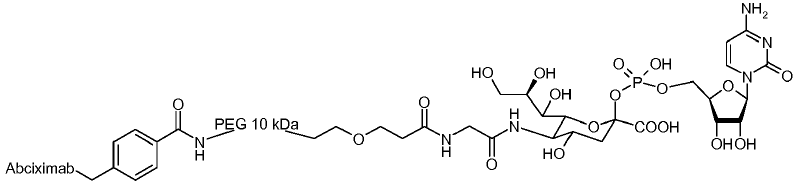

A solution of commercially available Abciximab (ProReo, 10 mg, 215 nmol, in a 2 mg/ml solution the commercial buffer) was placed in an Amicon ultracentrifugation device with a cut off of 10 kDa. Buffer consisting of 25 mM HEPES, which had been adjusted to pH 7.4 (5 ml) was added. An ultracentrifugation was performed at 4000 rpm at 10 °C for 10 min. Buffer consisting of 25 mM HEPES, which had been adjusted to pH 7.4 (10 ml) was added. An ultracentrifugation was performed at 4000 rpm at 10 °C for 10 min. Buffer consisting of 25 mM HEPES, which had been adjusted to pH 7.4 (10 ml) was added. An ultracentrifugation was performed at 4000 rpm at 10 °C for 10 min. The remaining solution of 0.65 ml was placed in a plastic reactor. Buffer consisting of 25 mM HEPES, which had been adjusted to pH 7.4 (3.85 ml) was added. A solution of *N*-((3-(ω-(4-formylbenzoylamino)10 kDa PEGyl)propionylamino)acetyl)-*O*²-[5']cytidylyl-ξ-neuraminic acid (14 mg, 1290 nmol) in a buffer consisting of 25 mM HEPES which had been adjusted to pH 7.0 (1.5 ml) was added. The reaction mixture was gently shaken at 300 rpm for 3 min at 20 °C. A freshly prepared 1.0 M solution of sodium cyanoborohydride in water (0.025 ml) was added. The reaction mixture was gently shaken at 300 rpm at 20 °C. After 1 h, another portion of the solution of sodium cyanoborohydride (0.025 ml) was added. After 1 h, another portion of the solution of sodium cyanoborohydrice (0.025 ml) was added. After 1 h, another portion of the solution of sodium cyanoborhydride was added. The solution was gently shaken at 300 rpm at 20 °C for 16 h. The reaction mixture was placed in an Amicon ultracentrifugation device with a cut off of 10 kDa. It was subjected to an ultracentrifiugation at 4000 rpm for 10 min at 18 oC. The remaining solution of 0.360 ml was filtered and subjected to a size exclusion chromatography on a Superdex200 gel with a bed size of 16 mm X 60 cm at a flow of 1 ml/min utilizing a buffer of 25 mM TRIS, 150 mM NaCl, which had been adjusted to pH 8.0 as eluent. Fractions containing the desired product were combined into two batches. Each of those batches were placed in an Amicon ultracentrifugation device with a cut off of 10 kDa. They were subjected to an ultracentrifugation at 4000 rpm at 10 °Cfor 10 min, yielding 2.67 mg and 3.27 mg respectively. For quantification a molar absorbance of 10.94 was used on a Nanodrop ® spectrophotometer. The analysis of the product by SDS-PAGE were in accordance with the expectation for a conjugate of *N*-((3-(ω-(4-formylbenzoylamino)10 kDa PEGyl)propionylamino)acetyl)-*O*²-[5']cytidylyl-ξ-neuraminic acid to one N-terminus of Abxicimab.

### Step 2:

### Conjugation of Abciximab to the O-glycan of FVIII

A buffer consisting of 20 mM histidine, 10 mM CaCl₂, 150 mM NaCl, 0.02% Tween80 and 1 M glycerol which had been adjusted to pH 7.35 (2.5 ml) was added to a solution of of B-domain deleted FVIII which has a residual B-domain sequence of SFSQNSRHPSQNPPVLKRHQR at the C-terminus of the heavy chain (5.7 mg/ml, 1 mg, 5.6 nmol) in a buffer consisting of 20 mM imidazole, 10 mM CaCl₂, 150 mM NaCl, 0.02% Tween80 and 1 M glycerol which had been adjusted to pH 7.35. A solution of the conjugate of of *N*-((3-(ω-(4-formylbenzoylamino)10 kDa PEGyl)propionylamino)acetyl)-*O*²-[5']cytidylyl-ξ-neuraminic acid to one N-terminus of Abxicimab (2.3 mg, 39.5 nmol) in a buffer consisting of 25 mM TRIS, 150 mM NaCl, which had been adjusted to pH 8.0 (0.323 ml) was added. The solution was placed in an Amicon ultracentrifugation device with a cut off of 10 kDa. It was subjected to a ultracentrifugation at 4000 rpm at 10 °C for 20 min. Buffer consisting of 20 mM histidine, 10 mM CaCl₂, 150 mM NaCl, 0.02% Tween80 and 1 M glycerol which had been adjusted to pH 7.35 (2 ml) was added. The solution was subjected to an ultracentrifugation at 4000 rpm at 10 °C for 30 min, leaving a solution with a volume of 1.4 ml. A solution of sialidase of A. Urifaciens (0.4 mg/ml, 242 U/mg, 0.0066 ml) and a solution of ST3Gal-I (2.5 mg/ml, 0.042 ml) were added subsequently. The reaction mixture was gently shaken at 32 °C for 15 min. After that the reaction mixture was left standing at 32 °C for 20.5 h. The reaction mixture was placed in an Amicon ultracentrifugation device with a cut off of 10 kDa. It was subjected to an ultracentrifugation at 4000 rpm at 10 °C for 15 min. The remaining solution of 0.300 ml was subjected to a size exclusion chromatography, using Superose 6 material with a bed size of 10 mm x 300 mm at a flow of 0.5 ml/min and using a buffer consisting of 10 mM Histidine, 1.7 mM CaCl₂, 0.01% Tween80, 0.3 M NaCl, 8.8 mM sucrose which had been adjusted to pH 7 as eluent. The fractions, containing the desired product were pooled and placed in an Amicon ultracentrifugation device with a cut off of 10 kDa. Buffer, consisting of 20 mM histidine, 10 mM CaCl2, 10% glycerol, 0.02% Tween80, 500 mM NaCl which had been adjusted to pH 6.07 (2.5 ml) was added. The solution was subjected to an ultracentrifugation at 4000 rpm at 10 °C for 15 min. Buffer, consisting of 20 mM histidine, 10 mM CaCl2, 10% glycerol, 0.02% Tween80, 500 mM NaCl which had been adjusted to pH 6.07 (1.5 ml) was added. The solution was subjected to an ultracentrifugation at 4000 rpm at 10 °C for 15 min. The remaining solution of 0.220 ml was placed in a plactic reactor. A solution of commercially available CMP NeuNAc (1.73 mg, 2800 nmol) in buffer consisting of 20 mM histidine, 10 mM CaCl2, 10% glycerol, 0.02% Tween80, 500 mM NaCl which had been adjusted to pH 6.07 (0.173 ml) was added. The reaction mixture was gently shaken at 300 rpm at 32 °C for 1 h. It was subjected to a size exclusion chromatography using using Superose 6 material with a bed size of 10 mm x 300 mm at a flow of 0.5 ml/min and using a buffer consisting of 10 mM Histidine, 1.7 mM CaCl₂, 0.01% Tween80, 0.3 M NaCl, 8.8 mM sucrose which had been adjusted to pH 7 as eluent. The fractions, containing the desired product were pooled an placed in an Amicon ultracentrifugation device with a cut off of 10 kDa. The pool was subjected to an ultracentrifugation at 4000 rpm at 10 °C for 5 min to give a solution of 0.275 ml of 0.0358 mg of a conjugation product of Abciximab to FVIII at the O-glycan. For quantification a molar absorbance of 13.15 was used on an Nanodrop ® spectrofphotometer. The SDS-PAGE analysis was in accordance with the expectation for a SDS-PAGE of a conjugation product of Abciximab to FVIII at the O-glycan.

## Claims

1. A recombinant Factor VIII molecule, wherein said molecule has prolonged circulatory half-life and reduced vWF binding capacity; wherein said molecule is covalently conjugated with at least one side group such that said side group is selected from one or more of the group consisting of: hydrophilic polymer, an albumin binder, an antibody or a fragment thereof, and albumin; and wherein the molecule comprises a point mutation and/or a deletion in the region spanning amino acid residues 1670-1684 in SEQ ID NO: 1.

2. A molecule according to any one of claim 1, wherein the Factor VIII molecule is a B-domain truncated variant.

3. A molecule according to claim 2, wherein a side group is covalently conjugated to the truncated B-domain, and wherein Factor VIII activation results in removal of the covalently conjugated side group.

4. A molecule according to claim 3, wherein said molecule is covalently conjugated with a hydrophilic polymer via an O-linked oligosaccharide in the truncated B domain, and wherein Factor VIII activation results in removal of the covalently conjugated hydrophilic polymer.

5. A molecule according to claim 4, wherein the O-linked oligosaccharide is attached to an O-glycosylation site that is made by truncation of the B-domain.

6. A molecule according to any one of claims 1-5, wherein said molecule comprises a point mutation in residue 1680.

7. A molecule according to any one of claims 1-5, wherein the Factor VIII molecule comprises a deletion of one or more amino acids in the region spanning residue 1670-1684.

8. A molecule according to any one of claim 1-7, wherein the side group is PEG.

9. A method of making a molecule according to any one of claims 1-8, wherein said method comprises attachment of a side group to a Factor VIII molecule having reduced capacity to bind vWF.

10. Use of a molecule according to any one of claims 1-8 for manufacture of a medicament for treatment of haemophilia.

11. A pharmaceutical composition comprising a molecule according to any one of claims 1-8.

## Patentansprüche

1. Rekombinantes Faktor-VIII-Molekül, wobei das Molekül über eine verlängerte zirkulierende Halbwertszeit und eine verminderte vWF-Bindungskapazität verfügt; wobei das Molekül kovalent mit mindestens einer Seitengruppe so konjugiert ist, dass die Seitengruppe aus einer oder mehreren aus der Gruppe bestehend aus den Folgenden ausgewählt ist: hydrophilem Polymer, einem Albuminbinder, einem Antikörper oder einem Fragment davon sowie Albumin; und wobei das Molekül eine Punktmutation und/oder eine Deletion in der Region, die sich über die Aminosäurereste 1670-1684 SEQ ID NO: 1 erstreckt, umfasst.

2. Molekül nach einem von Anspruch 1, wobei es sich bei dem Faktor-VIII-Molekül um eine in der B-Domäne verkürzte Variante handelt.

3. Molekül nach Anspruch 2, wobei eine Nebengruppe kovalent mit der verkürzten B-Domäne konjugiert ist und wobei die Aktivierung des Faktor VIII zur Entfernung der kovalent konjugierten Seitengruppe führt.

4. Molekül nach Anspruch 3, wobei das Molekül über ein über 0 verknüpftes Oligosaccharid in der verkürzten B-Domäne kovalent mit einem hydrophilen Polymer konjugiert ist und wobei die Aktivierung des Faktors VIII zur Entfernung des kovalent konjugierten hydrophilen Polymers führt.

5. Molekül nach Anspruch 4, wobei das über 0 verknüpfte Oligosaccharid an eine 0-Glykosylierungsstelle, die durch Verkürzung der B-Domäne entsteht, gebunden ist.

6. Molekül nach einem der Ansprüche 1-5, wobei das Molekül eine Punktmutation im Rest 1680 umfasst.

7. Molekül nach einem der Ansprüche 1-5, wobei das Faktor-VIII-Molekül eine Deletion von einer oder mehr Aminosäuren in der Region, die sich über die Reste 1670-1684 erstreckt, umfasst.

8. Molekül nach einem der Ansprüche 1-7, wobei es sich bei der Seitengruppe um PEG handelt.

9. Verfahren zur Herstellung eines Moleküls nach einem der Ansprüche 1-8, wobei das Verfahren das Binden einer Nebengruppe an ein Faktor-VIII-Molekül, das über eine verminderte Kapazität vWF zu binden, verfügt, umfasst.

10. Verwendung eines Moleküls nach einem der Ansprüche 1-8 zur Herstellung eines Arzneimittels für die Behandlung von Hämophilie.

11. Pharmazeutische Zusammensetzung, umfassend ein Molekül nach einem der Ansprüche 1-8.

## Revendications

1. Molécule de facteur VIII recombinante, ladite molécule ayant une demi-vie circulatoire prolongée et une capacité de liaison de vWF réduite ; ladite molécule étant conjuguée de façon covalente à au moins un groupe latéral de sorte que ledit groupe latéral soit choisi parmi l'un ou plusieurs du groupe constitué de : un polymère hydrophile, un lieur d'albumine, un anticorps ou un fragment de celui-ci, et l'albumine ; et la molécule comprenant une mutation ponctuelle et/ou une délétion dans la région couvrant les résidus d'acide aminé 1670 à 1684 dans SEQ ID NO: 1.

2. Molécule selon l'une quelconque de la revendication 1, la molécule de facteur VIII étant un variant tronqué du domaine B.

3. Molécule selon la revendication 2, dans laquelle un groupe latéral est conjugué de façon covalente au domaine B tronqué, et l'activation du facteur VIII conduit à l'élimination du groupe latéral conjugué de façon covalente.

4. Molécule selon la revendication 3, ladite molécule étant conjuguée de façon covalente à un polymère hydrophile par l'intermédiaire d'un oligosaccharide O-lié dans le domaine B tronqué, et l'activation du facteur VIII conduisant à l'élimination du polymère hydrophile conjugué de façon covalente.

5. Molécule selon la revendication 4, dans laquelle l'oligosaccharide O-lié est lié à un site de O-glycosylation qui est obtenu par troncature du domaine B.

6. Molécule selon l'une quelconque des revendications 1 à 5, ladite molécule comprenant une mutation ponctuelle au niveau du résidu 1680.

7. Molécule selon l'une quelconque des revendications 1 à 5, la molécule de facteur VIII comprenant une délétion d'un ou plusieurs acides aminés dans la région couvrant les résidus 1670 à 1684.

8. Molécule selon l'une quelconque des revendications 1 à 7, dans laquelle le groupe latéral est PEG.

9. Procédé de fabrication d'une molécule selon l'une quelconque des revendications 1 à 8, ledit procédé comprenant la liaison d'un groupe latéral à une molécule de facteur VIII ayant une capacité réduite à se lier à vWF.

10. Utilisation d'une molécule selon l'une quelconque des revendications 1 à 8 pour la fabrication d'un médicament pour le traitement de l'hémophilie.

11. Composition pharmaceutique comprenant une molécule selon l'une quelconque des revendications 1 à 8.
